# EUROPEAN PATENT APPLICATION

(11) **EP 2 987 488 A1**
(43) Date of publication of application: **24.02.2016**
(21) Application number: 15189194.2
(22) Date of filing: 28.03.2011
(51) Int. Cl.: A61K 31/426, A61P 11/00, A61P 15/08, A61P 17/00, A61P 17/06, A61P 35/04

(54) **DIPHENYL ETHER COMPOUNDS FOR THE TREATMENT OF LUNG DISORDERS**

(30) Priority: 23.02.2011 IN 525CH2011; 28.07.2010 IN 2140CH2010
(62) Divisional of application: 11720594.8
(71) Applicant: Orchid Chemicals and Pharmaceuticals Ltd, Nungambakkam 600 034 Chennai (IN)
(72) Inventor: NARAYANAN, Shridhar, 600 119 Tamil Nadu (IN); MOOKKAN, Jeyamurugan, 600 119 Tamil Nadu (IN); KULATHINGAL, Jayanarayan, 600 119 Chennai (IN); NARAYANAN, Surendran, 600 119 Chennai (IN); SINGH, Gajendra, 600 119 Chennai (IN); BALASUBRAMANIAN, Gopalan, 600 119 Chennai (IN)
(74) Representative: Duckett, Anthony Joseph

(57) **Abstract**

Described herein are compounds of formula (I), their derivatives, analogs, tautomeric forms, stereoisomers, polymorphs, hydrates, solvates, pharmaceutically acceptable salts and compositions, metabolites and prodrugs thereof, for use in treating liver diseases such as non-alcoholic fatty liver disease (NAFLD) and non-alcoholic steatohepatitis (NASH), and other fibrotic diseases of the liver; diabetic complications such as macro (ischemic heart disease, cerebrovascular disease and peripheral vascular disease) and micro (cataract, retinopathy nephropathy neuropathy, maculopathy and glaucoma) vascular complication; and cardiovascular diseases such as atherosclerosis, restenosis, hypertension, vasospasm, and cardiac hypertrophy; and lung disorders and lung fibrosis.

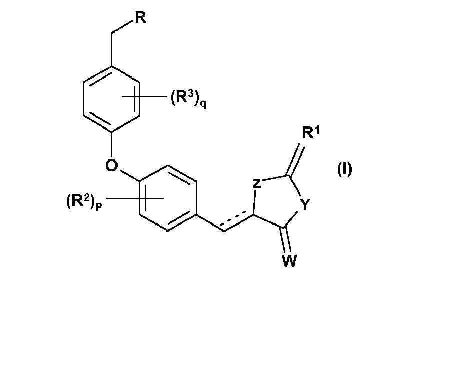

## Description

### Field

Described herein are compounds of formula (I) for use in prophylaxis and treatment of liver disorders such as non alcoholic fatty liver disease (NAFLD), non alcoholic steatohepatitis (NASH), alcoholic steatohepatitis (ASH); diabetic complications such as macro (ischemic heart disease, cerebrovascular disease and peripheral vascular disease) and micro (cataract, retinopathy, nephropathy, neuropathy, maculopathy and glaucoma) vascular complication; and cardiovascular diseases such as atherosclerosis, restenosis, hypertension, vasospasm and cardiac hypertrophy.

Disclosed herein are methods of treatment of NAFLD/NASH using the compound of formula (I), their derivatives, analogs, tautomeric forms, stereoisomers, polymorphs, hydrates, solvates, pharmaceutically acceptable salts, compositions, metabolites and prodrugs thereof.

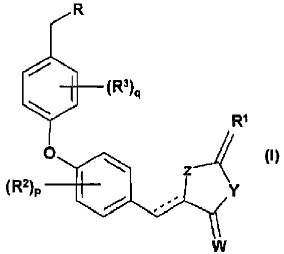

### Background

Hepatic steatosis, in non-alcoholics or people with no history of other known liver disease, is non-alcoholic fatty liver disease (NAFLD) and its progressive form is called non-alcoholic steatohepatitis (NASH) (J. Gastrointestin. Liver. Dis., 2007, 16, 39-46). NASH is characterized by insulin resistance, steatosis and necroinflammation with or without centrilobular fibrosis. There is no pharmacologic therapy that has conclusively shown to be an effective treatment for NASH (New England Journal of Medicine, 2006, 355, 2297-2307).

The pathogenesis of NASH is often conceptualized as a two-hit process, consisting of hepatic triglyceride accumulation (First hit), followed by the development of oxidative stress and cytokine expression leading to steatohepatitis (Second hit). Multiple metabolic processes can result in hepatocellular triglyceride accumulation including: (1) Excess dietary intake. Dietary triglycerides are delivered to the liver in the form of chylomicrons. In addition, dietary calories stored in adipose tissue as fat represent a source of fatty acids and triglycerides that can be delivered to the liver in the form of lipoprotein particles and free fatty acids (FFA). (2) Increased rates of lipogenesis resulting from the *de novo* synthesis of fatty acids and triglycerides in the liver. (3) Decreased rates of β-oxidation of fatty acids in the liver. (4) Decreased rates of export of cholesterol esters and triglycerides from the liver as very low density lipoprotein (VLDL). Insulin resistance is associated with increased lipolysis and reduced postprandial uptake and storage of fatty acids in adipose tissue, leading to increased fatty acid flux to the liver. In turn, increased liver fat content contributes to hepatic insulin resistance. Hyperinsulinemia induces sterol regulatory element-binding protein-1c (SREBP-1c) expression and hyperglycemia activates carbohydrate response element binding protein (ChREBP), both of which increase hepatic fatty acid synthesis *(*World J Gastroenterol, 2008, 14(1), 22-28).

Cytokines are attractive candidates for the 'second hit' in the pathogenesis of NASH. They are capable of producing all the classical histological features of NASH, including hepatocyte death/apoptosis (Tumor necrosis factor-α (TNF-*α*)), neutrophil chemotaxis (IL-8) and hepatic stellate cell activation (TNF-*α*, transforming growth factor-*β* (TGF-*β*)). In addition, it has been shown that patients with NASH have an increased expression of TNF-*α* and mRNA both in their liver and adipose tissue compared to obese controls, and this over-expression correlated with histological severity (Current science, 2006, 90(5), 10).

There is enough literature precedence to suggest a strong association of NAFLD with metabolic syndrome and that obesity and diabetes are considered to be the two major risk factors for the development of NAFLD. Patients with the metabolic syndrome and NAFLD present a higher risk of mortality from cardiovascular diseases compared with those without fatty liver (Nature Reviews Drug Discovery, 2009, 8, 361-367) and NAFLD is a predictor factor for the onset of type-2 diabetes. In these step-by-step multifactorial events, insulin and insulin resistance play a fundamental role. In fact, insulin resistance is present in approximately 98% of patients with NAFLD. On the basis of these patho-physiological events, treatment of NAFLD should regulate the metabolism of lipids in the muscle and adipose tissue and, therefore, reduce the entry of FFA in the liver through the correction of insulin resistance and/or modify the intra-hepatic metabolism of lipids and carbohydrates to prevent new synthesis of FFAs (Expert. Opin. Emerging. Drugs., 2008, 13, 1-14).

NAFLD presents a special challenge for several reasons. The exact prevalence of disease is unknown, however, its association with highly prevalent conditions (obesity, type 2 diabetes, dyslipidemia) suggests that a very high number of subjects may be at risk. NAFLD markers like alanine aminotransferase are not universally accepted, as progressive liver disease may also be present in subjects with normal enzyme levels. Old NAFLD patients may have multiple metabolic defects and their final prognosis is more severely regulated by the cardiovascular complications of the metabolic syndrome than by liver disease (Best Practice & Research Clinical Gastroenterology, 2004, 18, 1105-1116).

No specific drug is actually available to treat liver steatosis or NASH, though, there are reports on the human studies conducted by using anti-diabetic/insulin-sensitizing agent (Metformin, Pioglitazone and Rosiglitazone), anti-hyperlipidaemic agent (Clofibrate, Gemfibrozil, Atorvastatin, Pravastatin and Probucol), anti-obesity agent (Orlistat) and cytoprotective agent (Ursodeoxycholic acid, UDCA) (Expert. Opin. Emerging. Drugs. 2008, 13, 1-14). Clinical trials conducted with Rosiglitazone (for 48 weeks, 4 mg twice daily) showed significant improvement in hepatocellular ballooning, insulin sensitivity and mean serum aminotransferase levels, however, weight gain occurred in 67 % of the patients and a median weight gain increase of 7.3 *%* (Hepatology, 2003, 38, 1008-1017). Another pilot study was conducted with Pioglitazone in NASH patients, wherein the patients were treated with 30 mg of Pioglitazone daily for 48 weeks. Though there was improvement in histological features, the main side effects were weight gain and increased body adiposity *(*Hepatology 2004, 39, 188-196).

A randomized placebo-control study was carried out with Betaine (derived from the oxidation of dietary sources of choline) wherein the NASH patient received 20 g of Betaine daily for 12 months. This treatment, however, failed to improve the reduced serum antioxidant status, insulin resistance and adiponectin levels (Hepatology, 2009, 50(6), 1818-1826). A Pilot study involving Metformin was conducted (for 48 weeks, wherein the patient received 2 g/day). There was marked association between weight loss and improvement in NASH activity index. In addition, there was also improvement in insulin sensitivity, but it did not correlate with histological changes. The weight loss and improvements in Alanine aminotransferase (ALT) levels continued for the duration of therapy, however stopping the treatment followed promptly by weight gain and followed by increase in aminotransferase levels *(*Aliment Pharmacol Ther., 2008, 29, 172-192).

A preliminary clinical trials carried out with Atorvastatin (HMG-CoA reductase inhibitors) showed improvement in Total chlolesterol (TC) and Triglyceride (TG) levels that no other drug showed. This seems to be the first option in obese/dyslipidemic patients with NASH. But did not show significant improvement in blood glucose levels and body mass index (BMI). Another preliminary clinical trials with Pentoxifylline (PDE4 inhibitor) showed a fast and deep decrease of ALT and γ-glutamyl transpeptidase (GGT) after 10 weeks of treatment without interfering with other biochemical parameters, and this effect remained stable during the whole period of surveillance. But this also did not significantly improve the blood glucose levels and BMI (J Gastrointestin Liver Dis, 2007, 16, 39-46).

In a small pilot study, with Anti-obesity drug Orlistat (a reversible inhibitor of gastric and pancreatic lipase, block triglycerides absorption and promote weight loss) showed an improvement in aminotransferase, but changes in serum glucose and lipid profile was not statistically significant (Aliment Pharmacol Ther, 2004, 20, 623-628).

NAFLD has a high prevalence of about 14-30 % in the general population, involving all age groups and ethnicities, while the occurrence of NASH is about 3 %. Recent epidemiological studies in Italy have shown that the incidence of NAFLD is on the steady rise. NAFLD/NASH patients are mostly associated with obesity, diabetes and dyslipidemia (Expert Opinion on Emerging Drugs 2008, 13, 1-14). If any single compound has effect on all the three indications, i.e., obesity, diabetes and dyslipidemia, then it would be the right choice for NAFLD/NASH. Hence, the development of drug that would address all the three indications will be useful for the treatment of NAFLD/NASH.

NASH is also associated with patients using certain drugs. For instance, Tamoxifen is used worldwide as an antiestrogenic agent in patients with estrogen receptor positive early in breast cancer. Massive hepatic steatosis was observed in one-third of non-obese breast cancer patients as a result of exposure to tamoxifen and some of these patients even developed NASH (Internal Medicine, 2002, 41(5), 345-350).

NAFLD is widely common in individuals with mild or severe central obesity and subjects with type 2 diabetes or dyslipidemia (The Journal of Medicine, 2004, 62, 217). From the foregoing, it is now apparent that there is no specific drug for the treatment of NAFLD/NASH. Other drugs available in the market that were attempted to treat NAFLD/NASH suffered from side effects. It is now evident that there is an unmet need involved in the treatment of NAFLD/NASH and it is imperative to identify compounds that can appreciably treat NAFLD/NASH without showing undesirable/side effects as described above.

Progression of liver fibrosis has been found in a third of nonalcoholic steatohepatitis patients (Hepatology, 2004, 40, 820-826). This chronic disease cannot be treated successfully with conventional antifibrotic and anti-inflammatory drugs currently on the market, because they either lack efficacy or cause too many side effects. Antifibrotic agents targeting hepatic stellate cells (HSC) are considered as a promising strategy to increase their therapeutic potential. HSC represent 5-8 % of all human liver cells and 13 % of the volume of sinusoidal cells. Stellate cells are located in the perisinusoidal space of Disse beneath the endothelial barrier. Following acute or chronic liver injury, HSC are activated and undergo a process of trans-differentiation leading to a myofibroblastic phenotype. Upon stimulation the cells start trans-differentiating into activated stellate cells, which secrete fibrogenic factors including collagen in the liver. Collagen secretion is a remarkable property of activated HSC. Overproduction of collagen by activated HSC is a critical step in the development of liver fibrosis. It is not surprising, therefore, that activated HSCs are considered the major cellular target to prevent the progression of liver fibrosis. In fact, most of the antifibrotic treatments that are currently under evaluation are aimed at inhibiting the activation, proliferation or synthetic products of HSCs *(*J Gastroenterol, 2000, 35, 665-662). Fibrosis, characterized by the pathological accumulation of collagen, is increasingly recognized as an important feature of many chronic diseases and as said earlier, since the conventional antifibrotic and anti-inflammatory drugs available in the market that are used in the treatment of liver fibrosis either lack the required efficacy or show side effects, there is a need to develop drugs that address the above-mentioned disadvantages.

Diabetic complications can be divided into macro (ischemic heart disease, cerebrovascular disease and peripheral vascular disease) and micro (cataract, retinopathy, nephropathy, neuropathy, maculopathy and glaucoma) vascular complication and may be caused, for example, by accumulation of polyol (for example, sorbitol), free radical peroxidation and glucosylation of proteins at the site of lysine residues. Hyperglycemia causes several biochemical abnormalities that are thought to contribute to diabetic complications. Increased intracellular glucose concentrations activate aldose reductase, an enzyme that converts glucose to sorbitol. (The Journal of the American Medical Association, 2002, 288, 2579-88). The reduction of glucose to sorbitol is an energy-dependent process and excessive enzyme activity results in oxidative stress, which causes cellular dysfunction and damage. Glucose attaches non-enzymatically to proteins, resulting in the formation of advanced glycation end products that can cause cellular dysfunction and damage. Activation of protein kinase C (PKC), which plays a role in intracellular signaling, can alter gene expression and protein function, resulting in cell damage and dysfunction. The Aldose reductase pathway, advanced glycation end product pathway and PKC pathway are interrelated. All of these pathways are thought to contribute to retinal vascular endothelial cellular dysfunction and damage to abnormal cell growth and survival, increased vascular permeability, altered blood flow, ischemia, abnormal angiogenesis and to the basement matrix thickening associated with diabetic complications. These pathways are targets for therapies for diabetic complications.

Upregulation of proinflammatory nuclear transcription factors (Nuclear Factor-*k*B (NF-*k*B), Nuclear factor of activated T-cells (NFAT), Activator protein-1(AP-1)) can lead to the expression and production of inflammatory molecules such as inflammatory interleukins, growth factors, inducible nitric oxide synthase (iNOS) and proteolytic enzymes. These molecules have been linked to neovascularization in the cornea, suggesting that transcription factors mentioned could be suitable targets to inhibit angiogenic changes (Recent Prog. Horm. Res., 2001, 56, 239-263; J. Mol.Med., 2001, 79, 30-47; J. Clin. Invest., 2002, 110, 923-932; J. Endocrinol., 2001, 169, 453-459; J. Biol. Chem., 2000, 275, 4541-4544; J. Endocrinol., 2001, 169, 461-464). Recent study employing multiple sequence alignment using computational tools suggests (Medical Hypotheses, 2008, 70, 148-155) a close association between aldose reductase, Vascular endothelial growth factor (VEGF), Nitrous Oxide Synthase (NOS), Placental growth factor (PlGF), advanced glycation end products- receptor for AGE (AGE-RAGE), angiopoietins and cytokines in the pathogenesis of diabetic complications and hence, a multi-pronged approach will be effective in tackling this condition. Additionally, TNF-α is a serum marker for proliferative diabetic retinopathy (PDR) in Type I patients (J. Diab. Comp., 2008, 22 309-316) suggesting inhibition of lipopolysaccharide (LPS) induced TNF- α as a useful tool for treatment of diabetic complications.

In case of diabetic complications, the studies support the involvement of multiple mechanisms in the pathogenesis of the disease and hence targeting a single mechanism of action may not be effective in long-term treatment of diabetic complications. Thus it, necessitates that compounds for treating diabetic complications hit multiple targets including but not limited to aldose reductase, iNOS, TNF-α and IL-6 and hence there is a need for a new molecule which satisfies these stipulation.

US6794401B2 discloses the compounds of the formula (II), 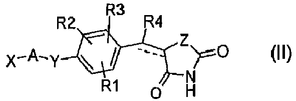 its tautomeric forms, its stereoisomers, its polymorphs, its pharmaceutically acceptable salts, its pharmaceutically acceptable solvates, wherein - - - represents an optional double bond; Y represents O, S or NR, wherein R represents hydrogen or alkyl; Z represents O or S; R₁, R₂, R₃ and R₄ may be same or different and independently represent hydrogen, halogen, hydroxy, nitro, cyano, formyl, amino, alkyl or alkoxy; A represents a bond or substituted or unsubstituted groups selected from aryl, heterocyclyl and heteroaryl ring; X represents an α-amino carboxylic acid or α-amino carboxylic acid derivative bonded to A or Y through its α-side chain.

US7521465B2 discloses the compounds of the formula (III), 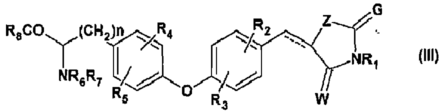 their analogs, their tautomeric forms, their stereoisomers, their polymorphs, their pharmaceutically acceptable salts, their pharmaceutically acceptable solvates, wherein ---- represents an optional bond; W represents O or S; Z represents CR₁₀, O or S; G represents O, S or together with R₁₀ forms a 5 or 6 membered aromatic or heteroaromatic ring system containing 1 or 2 heteroatoms selected from O, S or N; n represents 1. R₂, R₃, R₄ and R₅ are selected from hydrogen, halogens; hydroxy, nitro,

cyano, formyl, amino, substituted or unsubstituted groups selected from linear or branched (C₁-C₆) alkyl groups; (C₁-C₆) alkoxy groups. R₆ and R₇ may be same or different and independently represent H, substituted or unsubstituted groups selected from alkyl, alkenyl, aryl, heteroaryl, heterocyclyl and COR₁₂; wherein R₁₂ represents substituted or unsubstituted groups selected from alkyl, alkenyl, aryl, alkenyloxy, aryloxy, alkoxy, aralkyl and aralkoxy. R₈ represents -OR₁₃ or NR₁₄R₁₅, wherein R₁₃ represents hydrogen, substituted or unsubstituted groups selected from alkyl, alkenyl, aryl, aralkyl, heteroaryl and a counterion; R₁₄ and R₁₅ may be same or different and independently represent hydrogen, groups selected from alkyl, alkenyl and aryl. R₁ represents hydrogen, substituted or unsubstituted groups selected from alkyl, aryl, alkenyl, a counterion and -CH₂COOR; wherein, R represents H or (C₁-C₆) alkyl. R₁₀ optionally together with G forms a 5- or 6- membered aromatic or heteroaromatic ring system such as phenyl, furyl, pyrrolyl, pyridyl and the like.

US7781464B2 discloses the compounds of the formula (IV), 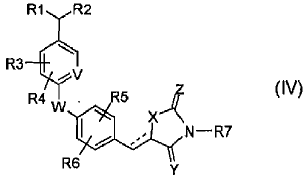 their pharmaceutically acceptable salts, wherein "----" represents an optional bond; V represents CH or N; Y represents O or S; W represents O or NR₈; R₈ is selected from hydrogen or substituted or unsubstituted linear or branched (C₁-C₆) alkyl groups; X represents CR₉, O or S; wherein R₉ is hydrogen or R₉ together with Z forms a 5 or 6-membered aromatic or heteroaromatic ring system containing 1 to 2 heteroatoms selected from O, S or N; Z represents O, S or together with R₉ forms a 5 to 6 membered aromatic or heteroaromatic ring system containing 1 to 2 hetero atoms selected from O, S or N; R₁ and R₂ may be same or different and are independently selected from hydrogen, halogen, hydroxy, nitro, cyano, formyl, amino, COR₁₀, substituted or unsubstituted groups selected from linear or branched (C₁-C₆) alkyl group and (C₁-C₆) alkoxy group; R₁₀ represents -OR₁₁ or NR₁₂R₁₃; where R₁₁, R₁₂ and R₁₃ are as defined therein; R₃, R₄, R₅ and R₆ are as defined therein; R₇ represents hydrogen, substituted or unsubstituted groups such as alkyl, alkenyl, -CH₂COOR, aryl group and a counter ion; wherein R represents H or (C₁-C₆) alkyl group.

Compounds of formula (II), (III) and (IV) are represented by the general formula (I).

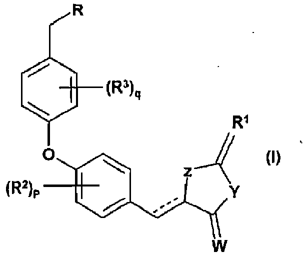

### Objective

Objective herein is to provide compounds of formula (I) for use in prophylaxis and treatment of liver disorders and associated diseases, fibrosis and diabetic complications such as macro (ischemic heart disease, cerebrovascular disease and peripheral vascular disease) and micro (cataract, retinopathy, nephropathy, neuropathy, maculopathy and glaucoma) vascular complication; comprising administering a therapeutically effective amount of compounds of formula (I).

Another objective herein are, methods of prophylaxis and treatment of liver disorders and associated diseases, fibrosis and diabetic complications such as macro (ischemic heart disease, cerebrovascular disease and peripheral vascular disease) and micro (cataract, retinopathy, nephropathy, neuropathy, maculopathy and glaucoma) vascular complication; comprising administering a therapeutically effective amount of compounds of formula (I).

Another objective is to provide compound of formula (I) for use in the treatment of liver disorder and associated diseases; lung disorder and associated diseases; diabetic complications and cardiovascular diseases; to provide compound of formula (I) for use in treatment of NAFLD (Non-Alcoholic Fatty Liver Disease), NASH (Non-Alcoholic Steatohepatitis), hepatic fibrosis, liver cirrhosis and alcoholic steatohepatitis; to provide a compound of formula (I) for use in the treatment of ischemic heart disease, cerebrovascular disease, peripheral vascular disease, cataract, retinopathy, nephropathy, neuropathy, maculopathy and glaucoma; to provide compound of formula (I) for use in the treatment of atherosclerosis, restenosis, hypertension, vasospasm, and cardiac hypertrophy; to compound of formula (I) for use in the treatment of psoriasis, lung metastasis, lung fibrosis, scleroderma, polycystic ovary syndrome and obstructive apnoea; to provide compound of formula (I) for use in the treatment of diabetic retinopathy, diabetic nephropathy, diabetic neuropathy and diabetic cataract; to provide compound of formula (I) for reducing plasma or liver triglycerides levels and reducing plasma glucose levels; to provide compound of formula (I) for inhibiting TNF-α, IL-6, iNOS, matrix metalloproteinase-2 or aldose reductase; to provide a pharmaceutical composition to treat the said diseases or at least to provide the public with a useful choice.

### Summary

Described herein is a method of treatment or use of the compounds of formula (I), their derivatives, analogs, tautomeric forms, stereoisomers, polymorphs, hydrates, solvates, pharmaceutically acceptable salts, pharmaceutically acceptable compositions, metabolites and prodrugs thereof, 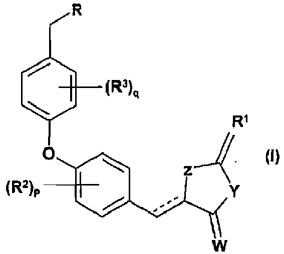 In particular, provided herein compounds of formula (I), their derivatives, analogs, tautomeric forms, stereoisomers, polymorphs, hydrates, solvates, pharmaceutically acceptable salts, pharmaceutically acceptable compositions, metabolites and prodrugs thereof, for use in treating liver disorders and associated diseases, lung disorder and associated diseases and diabetic complications such as macro (ischemic heart disease, cerebrovascular disease and peripheral vascular disease) and micro (cataract, retinopathy nephropathy, neuropathy, maculopathy and glaucoma) vascular complication, wherein ---- represents an optional bond; W represents O or S; Y represents NR⁴, S or O; wherein R⁴ represents hydrogen, substituted or unsubstituted groups selected from alkyl, alkenyl, aryl, a counter ion and -CH₂COR⁶; wherein R⁶ represents -OH, -NH₂, -NHOH or OR¹⁸; wherein R¹⁸ is an alkyl group; Z represents CR⁵ or S; R₁ represents O, S or together with R⁵ forms a fused 5 or 6 membered aromatic or heteroaromatic ring system containing carbon atoms or 1 or 2 heteroatoms selected from O, S or N;
R², R³ and R⁵ independently represent hydrogen, halogens, hydroxy, nitro, cyano, formyl, amino, alkyl, haloalkyl or alkoxy;
R represents either of U or V; wherein U represents hydrogen, halogen, hydroxy, nitro, cyano, formyl, amino, -COR¹⁰ or substituted or unsubstituted groups selected from linear or branched (C₁-C₆) alkyl group and (C₁-C₆) alkoxy group; R¹⁰ represents -OR¹¹ or -NR¹²R¹³; wherein R¹¹ represents hydrogen, a counter ion or substituted or unsubstituted groups selected from alkyl, alkenyl, aryl, aralkyl and heteroaryl; R¹² and R¹³ independently represent hydrogen or substituted or unsubstituted groups selected from alkyl, alkenyl, aryl and heteraryl; or R¹² and R¹³ together form a heteroaliphatic or heteroaromatic ring;
V represents 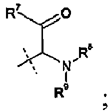 R⁷ represents -OR¹⁴; wherein R¹⁴ represents hydrogen, substituted or unsubstituted groups selected from alkyl, alkenyl, aryl, aralkyl, heteroaryl, a counter ion; and -NR¹⁵R¹⁶; wherein R¹⁵ and R¹⁶ independently represent hydrogen or substituted or unsubstituted groups selected from alkyl, alkenyl, aryl and heteroaryl; R⁸ and R⁹ independently represent hydrogen, -COR¹⁷ or substituted or unsubstituted groups selected from alkyl, alkenyl, aryl and heteroaryl; wherein R¹⁷ represents substituted or unsubstituted groups selected from alkyl, aryl, heteroaryl, alkenyl, alkenyloxy, aryloxy, alkoxy and aralkoxy;
p and q are integers are selected from 1-3.

In one preferred embodiment, described are compounds of formula (I) for use in treating NAFLD/NASH and NAFLD/NASH-related disorders and conditions comprising administering a therapeutically effective amount of compounds of formula (I).

In yet another embodiment, described are compounds of formula (I) for use in treating NAFLD/NASH comprising administering a therapeutically effective amount of compounds of formula (I).

In yet another embodiment described are compounds of formula (I) for use in treating NAFLD/NASH comprising administering a therapeutically effective amount of compound of formula (A).

In yet another embodiment, described are compounds of formula (I) for use in treating hepatic fibrosis and liver cirrhosis comprising administering a therapeutically effective amount of compounds of formula (I).

In yet another embodiment, described are compounds of formula (I) for use in diseases associated with NAFLD/NASH comprising administering a therapeutically effective amount of compounds of formula (I).

In yet another embodiment, described are compounds of formula (I) for use in treating cardiovascular diseases such as atherosclerosis, restenosis, hypertension, vasospasm, and cardiac hypertrophy.

In yet another embodiment, described are compounds of formula (I) for use in treating diabetic complications such as macro (ischemic heart disease, cerebrovascular disease and peripheral vascular disease) and micro (cataract, retinopathy, nephropathy, neuropathy, maculopathy and glaucoma) vascular complication.

In yet another embodiment, provided are compounds of formula (I) for use in treating lung disorders and associated diseases, lung fibrosis and lung metastasis.

In yet another embodiment, provided are compounds of formula (I) for use in treating psoriasis and scleroderma.

In yet another embodiment, provided are compounds of formula (I) having IL-6, Aldose reductase, iNOS, matrix metalloproteinase - 2 and TNF-α inhibiting activity.

In yet another embodiment, provided are compounds of formula (I) for reducing plasma or liver triglyceride level and plasma glucose levels.

### Brief description of the drawings

Figure 1: Effect of Compound of formula (A) on body weight in Diet Induced Obesity (DIO)/NAFLD mice.
Figure 2: Effect of compound of formula (A) on body weight in NAFLD/NASH mice.
Figure 3: Effect of Compound of formula (A) on liver weight in NAFLD/NASH mice.
Figure 4: Effect of Compound of formula (A) on liver TG level in NAFLD/NASH mice.
Figure 5: Effect of compound of formula (A) on glucose levels (fasted) in NAFLD/NASH mice.
Figure 6: Graphical representation of grading of effect of Compound of formula (A) on liver histopathology in NAFLD/NASH mice.
Figure 7(7a-7h): Effect of Compound of formula (A) on liver histopathological changes in NAFLD/NASH model.
Figure 8: Effect of compound of formula (A) on body weight gain in NAFLD/NASH mice.
Figure 9: Effect of Compound of formula (A) on glucose levels in NAFLD/NASH mice.
Figure 10: Effect of Compound of formula (A) on ALT levels in NAFLD/NASH mice.
Figure 11: Effect of compound of formula (A) on liver triglycerides in supranutritional diet induced NAFLD/NASH in C57BL/6 mice.
Figure 12: Effect of Compound of formula of (A) on hepatocellular vacuolation in supranutritional diet induced NAFLD/NASH mice.
Figure 13: Effect of Compound of formula (A) on liver histopathology in supranutritional diet induced NAFLD/NASH in C57BL/6 mice.
Figure 14: Effect of Compound of formula (A) on triglycerides levels in HC - HF (High Cholesterol - High Fat) diet fed Hamsters.
Figure 15: Effect of Compound of formula (A) on NEFA levels in HC-HF diet fed Hamsters.
Figure 16: Effect of compound of formula (A) on body weight gain in NAFLD/NASH mice.
Figure 17: Effect of compound of formula (A) on plasma Aspartate Aminotransferase (AST) in NAFLD/NASH mice.
Figure 18: Effect of compound of formula (A) on plasma ALT in NAFLD/NASH mice.
Figure 19: Effect of compound of formula (A) on hepatocellular ballooning in NAFLD/NASH mice.
Figure 20: Effect of compound of formula (A) on hepatic steatosis in NAFLD/NASH mice.
Figure 21 A: Effect of Compound of formula (A) on liver histopathology in supranutritional diet induced NAFLD/NASH in C57BL/6 mice Figure 22: Effect of compound of formula (A) on HOMA-IR in DIO mice.
Figure 23: Effect of compound of formula (A) on body weight gain in supranutritional diet induced NAFLD/NASH in nSTZ treated rats.
Figure 24: Graphical representation of grading of effect of compound of formula (A) on hepatic vacuolation (steatosis) in supranutritional diet induced NAFLD/NASH in nSTZ treated rats.
Figure 25: Effect of compound of formula (A) on hepatic vacuolation (steatosis) in supranutritional diet induced NAFLD/NASH in nSTZ treated rats.
Figure 26: Effect of compound of formula (A) on lung histopathology in bleomycin induced lung fibrosis in C57BL/6 mice.
Figure 27: Compound of formula (A) on lung histopathology (H&E staining) in bleomycin induced lung fibrosis in C57BL/6 mice.
Figure 28: Effect of compound of formula (A) on TNF-α level in lipopolysaccharides (LPS) challenged NAFLD/NASH mice.
Figure 29: Effect of disodium salt of Compound of formula (C) on plasma TG in HC - HF diet fed hamsters.
Figure 30: Effect of Compound of formula (A) and disodium salt of Compound of formula (C) on hepatocellular ballooning in supranutritional diet induced NAFLD/NASH mice.
Figure 31: Effect of Compound of formula (A) and Disodium salt of Compound of formula (C) on microvacoulation in supranutritional diet induced NAFLD/NASH mice.
Figure 32: Effect of Compound of formula (A) on liver histopathology in supranutritional diet induced NAFLD/NASH in C57BL/6 mice.
Figure 33: Effect of Compound of formula (A) disodium salts of Compound of formula of (C) on plasma TG in acute alcohol induced hyper-triglyceridemia in mice.
Figure 34: Effect of Compounds of formula (A), (B) and (C) on adipogenesis in 3T3-L1 mouse fibroblast.
Figure 35a & 35b: Collagen secretion estimation from HSC treated with Compounds of formula (B) and (C) using western blot technique.
Figure 36: Induction of apoptosis in HSCs by compounds of formula (B) and (C).
Figure 37: Induction of apoptosis by compounds of formula (B) and (C) is selective to HSCs
Figure 38: Inhibitory effect on iNOS production.
Figure 39: Serum TNF-α, Levels (pg/dL) after Oral Administration of Vehicle (n=5) and compound of formula (A) (50 mg/kg/day, n=4) in Male *ob*/*ob* Lean Control Mice for 10 days.
Figure 40: Serum IL-6 Levels (pg/dL) after Oral Administration of Vehicle (n=5) and compound of formula (A) (50 mg/kg/day, n=4) in Male *ob*/*ob* Lean Control Mice for 10 days.
Figure 41: Effect of Compound of formula (A) on Lenticular degeneration in STZ induced diabetic rat.

### Detailed description

Described herein are compounds of formula (I), 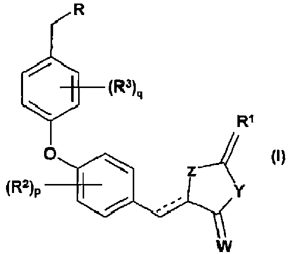 their derivatives, analogs, tautomeric forms, stereoisomers, polymorphs, hydrates, solvates, pharmaceutically acceptable salts, pharmaceutically acceptable compositions, metabolites and prodrugs for use in prophylaxis or treatment of liver disorders and associated diseases, fibrosis and diabetic complications such as macro (ischemic heart disease, cerebrovascular disease and peripheral vascular disease) and micro (cataract, retinopathy, nephropathy, neuropathy, maculopathy and glaucoma) vascular complication;
wherein, ---- represents optional bond; W represents O or S; Y represents NR⁴, S or O;
wherein R⁴ represents hydrogen or substituted or unsubstituted groups selected from linear or branched (C₁-C₆) alkyl groups such as methyl, ethyl, propyl, isopropyl, n-butyl, isobutyl, *t*-butyl and the like; alkenyl groups such as ethenyl, propenyl, butenyl and the like; aryl groups such as phenyl, naphthyl and the like; a counter ion is selected from alkali metals like Li, Na, K and the like or -CH₂COR⁶; wherein R⁶ represents -OH, -NH₂, -NHOH or -OR¹⁸; wherein R¹⁸ is an alkyl group; Z represents CR⁵, O or S; R¹ is selected from O, S or together with R⁵ forms a fused 5 or 6 membered aromatic or heteroaromatic ring system containing carbon atoms or 1 or 2 heteroatoms selected form O, S or N, such as phenyl, naphthyl, furyl, pyrrolyl, pyridyl and the like.

Suitable groups represented by R², R³ and R⁵ are selected from hydrogen, halogens such as fluorine, chlorine, bromine or iodine; hydroxy, nitro, cyano, formyl, amino, substituted or unsubstituted groups selected from linear or branched (C₁-C₄) alkyl groups such as methyl, ethyl, propyl, isopropyl, n-butyl, isobutyl, *t*-butyl and the like; haloalkyl groups selected from alkyl groups substituted by one, two, three or four halogen atoms such as chloromethyl, chloroethyl, trifluoromethyl, trifluoroethyl, dichloromethyl, dichloroethyl and the like; and (C₁-C₄) alkoxy groups such as methoxy, ethoxy, propoxy, butoxy and the like.

R represents either U or V; wherein U is selected from hydrogen, halogens such as fluorine, chlorine, bromine or iodine; hydroxy, nitro, cyano, formyl, amino, -COR¹⁰, substituted or unsubstituted groups selected from linear or branched (C₁-C₆) alkyl groups such as methyl, ethyl, propyl, isopropyl, n-butyl, isobutyl, t-butyl and the like; (C₁-C₆) alkoxy groups such as methoxy, ethoxy, propoxy, butoxy and the like; R¹⁰ represents -OR¹¹ or -NR¹²R¹³; where R¹¹ represents hydrogen, substituted or unsubstituted groups selected from (C₁-C₆) alkyl groups such as methyl, ethyl, propyl, isopropyl, n-butyl, isobutyl, t-butyl and the like; (C₂-C₂₀) alkenyl groups such as ethenyl, propenyl, butenyl and the like; aryl groups, including 5 to 14-membered mono-, bi- or tricyclic ring systems such as phenyl, naphthyl and the like; aralkyl groups such as benzyl, phenylethyl, phenylpropyl and the like; heteroaryl groups including 5 to 14-membered mono-, bi- or tricyclic ring systems such as pyridyl, thienyl, furyl, pyrrolyl, oxazolyl, thiazolyl, imidazolyl, isooxazolyl, oxadiazolyl, triazolyl, thiadiazolyl, tetrazolyl, pyrimidinyl, pyrazinyl, pyridazinyl, quinolinyl, dihydroquinolinyl, tetrahydroquinolinyl, isoquinolinyl, dihydroisoquinolinyl, tetrahydroisoquinolinyl and the like and or a counter ion such as sodium, potassium or magnesium;

R¹² and R¹³ may be same or different and independently represent hydrogen, substituted or unsubstituted groups selected from linear or branched (C₁-C₄) alkyl groups such as methyl, ethyl, n-propyl, isopropyl, n-butyl, isobutyl, *t*-butyl and the like; linear or branched (C₂-C₂₀) alkenyl groups such as ethenyl, propenyl, butenyl and the like; aryl groups such as phenyl, naphthyl and the like; heteroaryl groups such as pyridyl, thienyl, furyl, pyrrolyl, oxazolyl, thiazolyl, imidazolyl, isooxazolyl, oxadiazolyl, triazolyl, thiadiazolyl, tetrazolyl, pyrimidinyl, pyrazinyl, pyridazinyl, benzopyranyl, benzofuranyl, benzimidazolyl, benzoxazolyl, benzothiazolyl, benzopyrrolyl, benzoxadiazolyl, benzothiadiazolyl, benzodioxolyl, quinolinyl and the like or R¹² and R¹³ together form a heteroaliphatic or heteroaromatic ring such as a morpholinyl, piperidinyl, piperazinyl, pyrrolidinyl, azetidinyl, pyrrole, pyrazole, triazole and tetrazole and the like, which may be substituted;

V represents 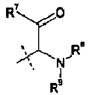 or its salts; R⁷ represents -OR¹⁴ or -NR¹⁵R¹⁶;

Suitable groups represented by R¹⁴ is selected from hydrogen, substituted or unsubstituted groups selected from linear or branched (C₁-C₄) alkyl groups such as methyl, ethyl, n-propyl, isopropyl, n-butyl, isobutyl, *t*-butyl and the like; linear or branched (C₂-C₄) alkenyl groups such as ethenyl, propenyl, butenyl and the like; aryl groups such as phenyl, naphthyl and the like; aralkyl groups such as benzyl, phenylethyl, phenylpropyl and the like; heteroaryl groups such as pyridyl, thienyl, furyl, pyrrolyl, oxazolyl, thiazolyl, imidazolyl, isooxazolyl, oxadiazolyl, triazolyl, thiadiazolyl, tetrazolyl, pyrimidinyl, pyrazinyl, pyridazinyl, benzopyranyl, benzofuranyl, benzimidazolyl, benzoxazolyl, benzothiazolyl, benzopyrrolyl, benzoxadiazolyl, benzothiadiazolyl, benzodioxolyl, quinolinyl and the like; the counter ion is selected from alkali metals like Li, Na, and K; alkaline earth metals like Ca and Mg; salts of different bases such as ammonium or substituted ammonium salts, diethanolamine, α-phenylethylamine, benzylamine, piperidine, morpholine, pyridine, hydroxyethylpyrrolidine, hydroxyethylpiperidine, choline aluminum, tromethamine and the like.

Suitable groups represented by R⁸ and R⁹ are selected from H, COR¹⁵, substituted or unsubstituted groups selected from linear or branched (C₁-C₄) alkyl groups such as methyl, ethyl, n-propyl, isopropyl, n-butyl, isobutyl, *t*-butyl and the like; linear or branched (C₂-C₄) alkenyl groups such as ethenyl, propenyl, butenyl and the like; aryl groups such as phenyl, naphthyl and the like and heteroaryl groups such as pyridyl, thienyl, furyl, pyrrolyl, oxazolyl, thiazolyl, imidazolyl, isooxazolyl, oxadiazolyl, triazolyl, thiadiazolyl, tetrazolyl, pyrimidinyl, pyrazinyl, pyridazinyl and the like.

Suitable groups represented by R¹⁵ and R¹⁶ are selected from hydrogen, substituted or unsubstituted groups selected from linear or branched (C₁-C₄) alkyl groups such as methyl, ethyl, n-propyl, isopropyl, n-butyl, isobutyl, *t*-butyl and the like; linear or branched (C₂-C₄) alkenyl groups such as ethenyl, propenyl, butenyl and the like; aryl groups such as phenyl, naphthyl and the like; heteroaryl groups such as pyridyl, thienyl, furyl, pyrrolyl, oxazolyl, thiazolyl, imidazolyl, isooxazolyl, oxadiazolyl, triazolyl, thiadiazolyl, tetrazolyl, pyrimidinyl, pyrazinyl, pyridazinyl, benzopyranyl, benzofuranyl, benzimidazolyl, benzoxazolyl, benzothiazolyl, benzopyrrolyl, benzoxadiazolyl, benzothiadiazolyl, benzodioxolyl, quinolinyl and the like; alkoxy groups such as methoxy, ethoxy, n-propoxy, isopropoxy and the like; aralkoxy groups such as phenylmethoxy, phenylethoxy, phenylpropoxy and the like and aryloxy groups such as phenoxy, napthoxy and the like.

when substituted the substituents may include without limitations, one or more substituents selected from halogens such as fluorine, chlorine, bromine or iodine; hydroxy, nitro, cyano, azido, nitroso, amino, hydrazine, hydrazide, hydroxamate, formyl, alkyl, haloalkyl, haloalkoxy, cycloalkyl, aryl, alkoxy, aryloxy, acyl, acyloxy, acyloxyacyl, heterocyclyl, heteroaryl, monoalkylamino, dialkylamino, acylamino, alkylaminocarbonyl, alkoxycarbonyl, aryloxycarbonyl, heterocyclylcarbonyl, arylsulfonyl, alkylsulfinyl, arylsulfinyl, alkylthio, arylthio, sulfamoyl, alkoxyalkyl groups and carboxylic acids and its derivatives such as carboxamide and carboxamidoalkyl.

p and q are integers and are selected from 1-3.

Provided herein are, compounds of formula (I) for use in treating liver disorders, lung disorders, diabetic complications, cardiovascular and associated diseases comprising administering compound of formula (I).

Described herein, compounds of formula (I) for use in treating NAFLD/NASH and NAFLD/NASH-related disorders and conditions comprising administering a therapeutically effective amount of compounds of formula (I).

Further described herein, compounds of formula (I) for use in treating NAFLD/NASH comprising administering a therapeutically effective amount of compounds of formula (I).

Further provided herein are, compounds of formula (I) for use in treating NAFLD/NASH comprising administering a therapeutically effective amount of compound of formula (A).

Hepatic fatty acid triglyceride and phospholipids synthesis are accelerated after acute and chronic ethanol consumption. Enhanced lipogenesis is a reflection of higher expression of lipogenic enzymes including fatty acid synthase, acyl CoA carboxylase (ACC), ATP citrate lyase (ACL), stearoyl CoA desaturase and malic enzyme, thereby enhancing fat accumulation. These enzymes are encoded by genes regulated by the transcription factor, sterol regulatory element binding protein-1 (SREBP-1). Individuals with alcohol-induced steatosis are vulnerable to developing alcoholic steatohepatitis (ASH), hepatic fibrosis, cirrhosis and even hepatocellular carcinoma (World J Gastroenterol 2007, 13, 4974-4978).

Further described herein, are compounds of formula (I) for use in treating hepatic fibrosis, alcoholic steatohepatitis (ASH), liver cirrhosis, hepatocellular carcinoma, lung disorder, lung fibrosis, lung metastasis and psoriasis.

NAFLD has been closely associated with classical cardiovascular risk factors, polycystic ovary syndrome (PCOS) and obstructive sleep apnoea (OSA) (Journal of Hepatology, 2008, 48, S104-S112).

Further described herein, are compounds of formula (I) for use in treating associated diseases such as polycystic ovary syndrome, obstructive apnoea, scleroderma psoriasis with NAFLD/NASH.

Further described herein, are compounds of formula (I) for use in treating lung disorders and associated diseases such as lung fibrosis and lung metastasis.

Further described herein, are compounds of formula (I) for use in treating psoriasis.

Further described herein, are compounds of formula (I) for inhibiting TNF-α, IL-6, iNOS, Aldose reductase and Matrix metalloproteinase-2.

Further described herein, are compounds of formula (I) for reducing plasma or liver triglycerides level and plasma glucose levels.

The term "analog" includes a compound, which differs from the parent structure by one or more C, N, O or S atoms. Hence, a compound in which one of the N atoms in the parent structure is replaced by an S atom is an analog of the former.

The term "stereoisomer" includes isomers that differ from one another in the way the atoms are arranged in space, but whose chemical formulae and structures are otherwise identical. Stereoisomers include enantiomers and diastereoisomers.

The term "tautomers" include readily interconvertible isomeric forms of a compound in equilibrium. The keto-enol tautomerism is an example.

The term "polymorphs" include crystallographically distinct forms of compounds with chemically identical structures.

The term "pharmaceutically acceptable solvates" includes combinations of solvent molecules with molecules or ions of the solute compound.

The term "derivative" refers to a compound obtained from a compound according to formula (I), an analog, tautomeric form, stereoisomer, polymorph, hydrate, pharmaceutically acceptable salt or pharmaceutically acceptable solvate thereof, by a simple chemical process converting one or more functional groups, such as, by oxidation, hydrogenation, alkylation, esterification, halogenation and the like.

Pharmaceutically acceptable salts of the present invention include alkali metals such as Li, Na, K and the like; alkaline earth metal such as Ca, Mg and the like; salts of organic bases such as diethanolamine, α-phenylethylamine, benzylamine, piperidine, morpholine, pyridine, hydroxyethylpyrrolidine, hydroxyethylpiperidine, choline and the like, ammonium or substituted ammonium salts, aluminum salts. Salts also include amino acid salts such as glycine, alanine, cystine, cysteine, lysine, arginine, phenylalanine, guanidine etc. Salts may include acid addition salts where appropriate which are sulphates, nitrates, phosphates, perchlorates, borates, hydrohalides, acetates, tartrates, maleates, citrates, succinates, palmoates, methanesulphonates, tosylates, benzoates, salicylates, hydroxynaphthoates, benzenesulfonates, trifluoroacetates, ascorbates, glycerophosphates, ketoglutarates and the like. Pharmaceutically acceptable solvates may be hydrates or comprising other solvents of crystallization such as alcohols.

A term once described, the same meaning applies for it, throughout the patent. Particularly preferred compounds of the present invention include:
1. Methyl (2*S*)-2-amino-3-(4-{4-[(2,4-dioxo-1,3-thiazolidin-5-yl)methyl] phenoxy}phenyl)propanoate or its salt;
2. (2*S*)-2-Amino-3-(4-{4-[(2,4-dioxo-1,3-thiazolidin-5-yl)methyl]phenoxy} phenyl)propanoic acid or its salt and
3. (4-{4-[(2,4-Dioxo-1,3-thiazolidin-5-yl)methyl]phenoxy}phenyl)acetic acid or its salt.

The pharmaceutically acceptable salts are prepared by reacting the compound of formula (I) with 1 to 10 equivalents of a base such as sodium hydroxide, sodium methoxide, sodium hydride, potassium t-butoxide, calcium hydroxide, magnesium hydroxide and the like, in solvents like ether, tetrahydrofuran, methanol, *t*-blitanol, dioxane, isopropanol, ethanol etc. Mixture of solvents may also be used. Organic bases such as diethanolamine, α-phenylethylamine, benzylamine, piperidine, morpholine, pyridine, hydroxyethylpyrrolidine, hydroxyethylpiperidine, choline, guanidine, ammonium, substituted ammonium salts and aluminum salts and amino acids such as glycine, alanine, cystine, cysteine, lysine, arginine, phenylalanine etc may be used for the preparation of amino acid salts. Alternatively, acid addition salts wherever applicable are prepared by treatment with acids such as hydrochloric acid, hydrobromic acid, nitric acid, sulfuric acid, phosphoric acid, *p*-toluenesulphonic acid, methanesulfonic acid, acetic acid, citric acid, maleic acid, salicylic acid, hydroxynaphthoic acid, ascorbic acid, palmitic acid, succinic acid, benzoic acid, benzenesulfonic acid, tartaric acid, oxalic acid and the like in solvents like ethyl acetate, ether, alcohols, acetone, tetrahydrofuran, dioxane etc. Mixture of solvents may also be used.

It should be noted that compounds of the invention may contain groups that may exist in tautomeric forms, and though one form is named, described, displayed and/or claimed herein, all the forms are intended to be inherently included in such name, description, display and/or claim.

The stereoisomers of the compounds forming part of this invention may be prepared by using reactants in their single enantiomeric form, in the process wherever possible or by conducting the reaction in the presence of reagents or catalysts in their single enantiomeric form or by resolving the mixture of stereoisomers by conventional methods. Some of the preferred methods include use of microbial resolution, resolving the diastereomeric salts formed with chiral acids such as mandelic acid, camphorsulfonic acid, tartaric acid, lactic acid and the like, wherever applicable or by using chiral bases such as brucine, cinchona alkaloids, their derivatives and the like.

Prodrugs of the compounds of formula (I) are also contemplated by this invention. A prodrug is an active or inactive compound that is modified chemically through *in-vivo* physiological action, such as hydrolysis, metabolism and the like, into a compound of this invention following administration of the prodrug to a patient. The suitability and techniques involved in making, using prodrugs are well known by those skilled in the art.

Various polymorphs of the compounds of the general formula (I), forming part of this invention may be prepared by crystallization of the compounds of formula (I) under different conditions. For example, using different commonly used solvents, or their mixtures for recrystallization; crystallizations at different temperatures; various modes of cooling, ranging from very fast to very slow cooling during crystallizations. Heating or melting the compounds followed by cooling gradually or immediately, one can also obtain polymorphs. The presence of polymorphs may be determined by solid probe NMR spectroscopy, IR spectroscopy, Raman spectroscopy, differential scanning calorimetry and powder X-ray diffraction or other such techniques.

Pharmaceutically acceptable solvates of the compounds of the formula (I) forming part of this invention may be prepared by conventional methods such as dissolving the compounds of the formula (I) in solvents such as water, methanol, ethanol, mixture of solvents such as acetone/water, dioxane/water, *N, N-*dimethylformamide/water and the like, preferably water and recrystallization by using different crystallization techniques.

The present invention also provides a pharmaceutical composition, containing one or more of the compounds of the general formula (I) as defined above, their derivatives, analogs, tautomeric forms, stereoisomers, polymorphs, hydrates, metabolites, prodrugs, pharmaceutically acceptable salts, pharmaceutically acceptable solvates in combination with the usual pharmaceutically employed carriers, diluents and the like, useful for the treatment of and/or prophylaxis of liver disorders such as NASH/NAFLD, hepatic fibrosis, liver cirrhosis, steatohepatitis and the like and associated diseases like cardiovascular disease, polycystic ovary syndrome, obstructive apnoea and the like; psoriasis; lung disorders such as lung fibrosis and the like and associated diseases such as lung metastasis and the like; and diabetic complications such as diabetic retinopathy, diabetic nephropathy, diabetic neuropathy, diabetic cataract and the like.

The pharmaceutical composition may be in the forms normally employed, such as tablets, capsules, powders, syrups, solutions, suspensions and the like, may contain flavorants, sweeteners etc. in suitable solid or liquid carriers or diluents, or in suitable sterile media to form injectable solutions or suspensions. The compositions may be prepared by processes known in the art. The amount of the active ingredient in the composition may be less than 70% by weight. Such compositions typically contain from 1 to 25%, preferably 1-15% by weight of active compound, the remainder of the composition being pharmaceutically acceptable carriers, diluents, excipients or solvents. Suitable pharmaceutically acceptable carriers include solid fillers or diluents and sterile aqueous or organic solutions.

Generally, the effective dose for treating a particular condition in a patient may be readily determined and adjusted by the physician during treatment to alleviate the symptoms or indications of the condition or disease. Generally, a daily dose of active compound in the range of about 0.01 to 1000 mg/kg of body weight is appropriate for administration to obtain effective results. The daily dose may be administered in a single dose or divided into several doses. In some cases, depending upon the individual response, it may be necessary to deviate upwards or downwards from the initially prescribed daily dose. Typical pharmaceutical preparations normally contain from about 0.2 - 500 mg of active compound of formula I and/or its pharmaceutically active salts or solvates per dose.

While the compounds of the invention can be administered as the sole active pharmaceutical agent, they can also be used in combination with one or more compounds of the invention or other therapeutic agents. When administered as a combination, the therapeutic agents can be formulated as separate compositions that are given at the same time or different times, or the therapeutic agents can be given as a single composition.

The term "therapeutically effective amount" or "effective amount" refers to that amount of a compound or mixture of compounds of formula (I) that is sufficient to effect treatment, as defined below, when administered alone or in combination with other therapies to a mammal in need of such treatment.

The term "animal" as used herein is meant to include all mammals, and in particular humans. Such animals are also referred to herein as subjects or patients in need of treatment. The therapeutically effective amount will vary depending upon the subject and disease condition being treated, the weight and age of the subject, the severity of the disease condition, the particular compound of formula (I) chosen, the dosing regimen to be followed, timing of administration, the manner of administration and the like, all of which can readily be determined by one of ordinary skill in the art.

The term "Hepatitis" as described herein refers to inflammation of liver. The term "Hepatic Steatosis" as described herein refers to accumulation of fat droplets or triglycerides in the cytoplasm of liver cells/ hepatocytes. The term "Hepatocyte Vacuolation" as described herein refers to liver cells/ hepatocytes containing vacuoles of various sizes.

The term "Hepatocellular ballooning" as described herein refers to a special form of liver cell degeneration associated with cell swelling and enlargement found particularly in steatohepatitis.

The term "Diabetic complications" includes but not limited to micro and macro vascular complications and refers to the diseases induced by diabetes (hyperglycemia).

The term "Cardiovascular disease" refers to the diseases of the heart and circulatory system.

The term "prophylaxis" or "prevention" means preventing the disease, ie, causing the clinical symptoms of the disease not to develop.

The term "treatment"/"treating" means any treatment of a disease in a mammal, including: (a) Inhibiting the disease, ie, slowing or arresting the development of clinical symptoms; and/or (b) Relieving the disease, ie, causing the regression of clinical symptoms.

The term "compound(s) for use" as used herein embrace any one or more of the following: (1) use of compound(s), (2) method of use of compound(s), (3) use in the treatment of, (4) the use for the manufacture of pharmaceutical composition / medicament for treatment/treating or (5) method of treatment / treating/ preventing / reducing / inhibiting comprising administering an effective amount of the active compound to a subject in need thereof.

From the foregoing description, one skilled in the art can easily ascertain the essential characteristics of this invention, and without departing from the spirit and scope thereof, make various changes and modifications of the invention to adapt it to various usages and conditions.

The present invention is provided by the examples given below, which are provided by the way of illustration only, and should not be construed to limit the scope of the invention. Variation and changes, that are obvious to one skilled in the art, are intended to be within the scope and nature of the invention.

Compounds falling within the scope of the invention are provided below in the Table 1.

**Table 1.**

| | |
|---|---|
| Compound of formula (A) | Compound of formula (B) |
| 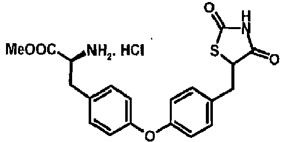 | 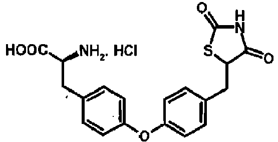 |
| Hydrochloride salt of Methyl (2*S*)-2-amino-3-(4-{4-[(2,4-dioxo-1,3-thiazolidin-5-yl)methyl]phenoxy}phenyl)propanoate | Hydrochloride salt of (2*S*)-2-Amino-3-(4-{4-[(2,4-dioxo-1,3-thiazolidin-5-yl)methyl]phenoxy}phenyl)propanoic acid |
| Compound of formula (C) | Di sodium salt of Compound of formula (C) |
| 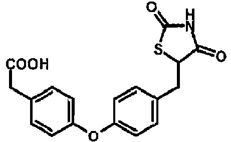 | 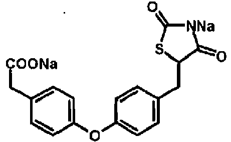 |
| (4-{4-[(2,4-Dioxo-1,3-thiazolidin-5-yl)methyl]phenoxy}phenyl)acetic acid | Disodium salt of (4-{4-[(2,4-Dioxo-1,3-thiazolidin-5-yl)methyl]phenoxy}phenyl)acetic acid |

The compound of formulae (I) and (A)-(C) or its salts can be prepared by methods described in US6794401B2, US 7521465B2 and US7781464B2.

### Biology Testing

### 1. NAFLD/NASH

To investigate the effect of compound of formulae (A), (B), (C) and their salts on key features of NAFLD/NASH, the relevant animal models were developed using supranutrional diet, bleomycin and lipopolysaccharides (LPS).

### Experiment - 1: Effect of Compound of formula (A) on body weight in DIO/NAFLD mice

A rodent obese and NAFLD model was developed by feeding supranutritional diet, which simulates several features of human obesity and NAFLD. These features were treated as mentioned below. Thirty C57BL/6 male mice aged at 6-8 weeks, body weight ranges between 16- 22 g were used for this study. Animals were divided into two groups. First group (n = 8, where n refers to number of animals) was treated as a normal control fed with chow feed (Nutri Lab® Rodent, Tetragon Chemie Pvt.Ltd., Bangalore) and the second group (n = 20) was treated with 60 Kcal % high fat diet (HFD) (Research Diets, New Brunswick, NJ, Cat# D 12492 with blue dye) for 90 days. Animals were selected based on their body weight. The Animals fed with normal chow diet (n=8) served as normal control (Group I) and animals fed with HFD were divided into two groups as DIO/NAFLD control (Group II) (n=10), and Group III (n=9) treated with compound formula (A) at 100 mg/kg for 28 days. During this period, animal body weight and feed intake was recorded daily.

### Experiment - 2: Effect of Compound of formula (A) in NAFLD/NASH mice

A rodent NAFLD/NASH model was developed by feeding supranutritional diet which simulates several features of human NAFLD/NASH. These features were treated as mentioned below. Fifty C57BL/6 male mice aged 6-8 weeks, and body weight ranges between 16- 36 g were used for this study. Animals were divided into two groups, first group (n=10) treated as a normal control, was fed with chow feed (Nutri Lab® Rodent, Tetragon Chemie Pvt.Ltd., Bangalore) and the second group (n=40) fed with 60 Kcal% HFD (Research Diets, New Brunswick, NJ, Cat# D12492 with blue dye) and 40 % high fructose liquid (HFL) for 60 days. After 45 days, two animals from disease induction and normal control groups were sacrificed; liver histopathology examination was carried out to confirm the induction of NAFLD/NASH. Animals fed with normal chow diet served as a normal control (Group I) and animals fed HFD and HFL were divided into three groups (n=6-8) as NAFLD/NASH control (Group II), Group III and Group IV were dosed with compound of formula (A) and Metformin at 100 and 350 mg/kg p.o respectively for 28 days. On 28th day, animals were kept 12 hours fasting and 29^{th} day blood was collected, plasma separated and used for biochemical and compound concentration analysis. The animals were then sacrificed. Liver was collected and weighed. Liver tissues were used for compound concentration analysis, triglycerides estimation and histopathology examination. Gross pathology of liver was also observed.

### Experiment -3: Effect of Compound of formula (A) in NAFLD/NASH mice

The experiment - 2 protocol was followed. There compound of formula (A) was compared with other antidiabetic compounds (Figures - 8-13).

### Experiment - 4: Effect of Compound of formula (A) in High cholesterol-high fat diet (HC - HF) diet fed Hamsters

Dyslipidemia in hamsters were developed by feeding supranutritional diet with fructose liquid (10 %) which simulates features of dyslipidemia in human. These features were treated as mentioned below. Thirty male Golden Syrian hamsters aged 14-16 weeks, body weight ranges between 100- 160 g were used for this study. Animals were divided into two groups. First group (n = 7) was treated as a normal control and was fed with chow feed (Nutri Lab® Rodent, Tetragon Chemie Pvt.Ltd., Bangalore) and the second group (n = 23) was fed with HC-HF diet (in-house made), the composition of which were Corn oil 11.50 %, Coconut oil 11.50 %, Cholesterol 0.50 %, Sodium cholate 0.25 % and Nutri Lab® feed 76.25 % and animals were provided with 10% fructose in the drinking water. After 14 days, animals were bled for estimation of the biochemical parameter *viz.,* triglycerides (TG), total cholesterol (TC) and glucose to confirm induction of disease. Based on the biochemical parameter, animals were grouped. Animals fed with normal chow diet (n=7) served as normal control (Group I) and animals fed supranutritional diet with fructose liquid (10 %) were divided into three groups, as disease control (Group II) (n=7), Group III (n=8) and Group IV (n=8) treated with compound of formula (A) at 30 mg/kg and 100 mg/kg p.o respectively for 28 days. During this period, animal body weight was recorded daily. Animals were kept for 4 hours fasting and bled on 16^{th} and 28^{th} days of treatment, plasma and serum were separated and used for biochemical analysis viz., TG, TC, plasma glucose and Non-esterified fatty acids (NEFA).

### Experiment-5: Dose Response of Compound of formula (A) on NAFLD/NASH in C57BL/6 mice

To develop NAFLD/NASH model, the same protocol was followed as mentioned in Experiment - 2. At the end of induction period the animals were grouped as follows. Animals fed with normal chow diet (n=9) served as Normal control (Group 1) and animals fed HFD and HFL were divided into four groups (n=9-12) as NAFLD/NASH control (Group II), Groups III, IV, and V were treated with compound of formula (A) at 10, 30 and 100 mg/kg p.o. respectively for 28 days. After 28 days of treatment animals were kept for 12 hours fasting and blood was collected for biochemical estimations. Following blood collection, animals were sacrificed, liver was collected and weighed. Liver tissues were used for compound concentration analysis, TG estimation and histopathological examination. Gross pathology of liver was also observed.

### Experiment-6: Effect of compound of formula (A) on Insulin Tolerance Test (ITT) in Diet Induced Obese (C57BL/6) mice

The animal model with obesity with insulin resistance was developed by feeding 60 Kcal% high fat diet, which simulates several features of human obesity and insulin resistance. These features were treated as mentioned below. Thirty C57BL/6 male mice aged 6-8 weeks, and body weight ranges between 16-22 g were used. Animals were divided into two groups. First group (n=10) was treated as a normal control fed chow feed (Nutri Lab® Rodent, Tetragon Chemie Pvt.Ltd., Bangalore) and the second group (n =25) fed 60 Kcal % high fat diet (HFD) (Research Diets, New Brunswick, NJ, Cat# D12492 with blue dye) for 120 days. At the end of induction period the animals were selected based on insulin resistance for further study. Animals fed with normal chow diet (n=10) served as normal control (Group I) and animals fed HFD were further divided into two groups (n=6-7) as DIO control (Group II) and Group III treated with compound of formula (A) at 30 mg/kg for 28 days. During this period, body weight and feed intake was recorded daily. On 28^{th} day, animals were subjected to 12 hours fasting and ITT was performed. After a span of 3 days, once again animals were subjected to 12 hours fasting, blood was collected, and plasma or serum was separated for biochemical analysis.

### Experiment-7: Dose response of compound of formula (A) on supranutritional diet induced NAFLD/NASH in neonatal streptozotocin (nSTZ) treated rats

A rodent NAFLD/NASH model was developed by feeding supranutritional diet to nSTZ treated rats. This model simulates several features of human NAFLD/NASH. One-day-old male Wistar rat pups were injected either streptozotocin (STZ) 100 mg/kg or normal saline (i.p.) to develop insulin resistance. Twenty-one days post treatment STZ treated animals were divided into two groups and fed either regular chow diet or 60 Kcal % high fat diet with 40% fructose in drinking water for three months to induce NAFLD/NASH. The saline treated animals were fed regular chow diet. After 45 days and 60 days of induction one animal from induction group was sacrificed, liver histopathology examination was carried out to confirm the induction of NAFLD/NASH. After 90 days of induction period, the animals were divided into following groups. The animals were fed with regular chow diet kept as Normal control (Group I, n=9) and nSTZ control (Group II, n=7). The disease induction groups were further divided into four groups NAFLD/NASH control (Group-III, n=9) and Group IV (n=7), Group V (n=8) and Group VI (n=9) treated Compound of formula (A) at 1, 3 and 10 mg/kg body weight p.o. respectively for 48 days. Daily body weight, feed and water intake were recorded through out the experimental period. On 31^{st} day and 48^{th} day, animals were kept 12 hours fasting and 32^{nd} day and 49^{th} day blood was collected, plasma separated and used for biochemical and compound concentration analysis. On 49^{th} day, after blood collection the animals were sacrificed. Liver and fat pads were collected and weighed. Liver tissues were used for compound concentration analysis, TG estimation, and histopathology examination and expression study. Gross pathology of liver was also observed.

### Experiment-8: Effect of compound of formula (A) on Bleomycin induced lung fibrosis in C57BL/6 mice (Prophylactic)

Female C57BL/6 mice were used in this study. Animals were divided into four groups based on their body weight. To induce fibrosis bleomycin was administered at 0.1 U/animal (volume 50 µL) intratracheally to Group II (Disease control), Group III and Group IV, whereas Group I (Normal control) received only saline. The bleomycin challenged Group III and Group IV animals were treated with pentoxifylline (20 mg/kg p.o.) and compound of formula (A) (100 mg/kg p.o.) respectively, whereas Group I and the bleomycin challenged Group II animals received vehicle for 7 days before and 14 days after bleomycin administration. Body weight, feed and liquid intake and mortality rate were recorded. At the end of the study, animals were bled and plasma was stored in deep freezer (-80 °C) for biochemical parameters estimation. Then, the animals were sacrificed and lung tissue harvested and weighed. Right lung tissue was stored in deep freezer (-80 °C) for expression study and drug concentration analysis. The left lung tissues was fixed in formalin (10 %) by slow infusion, then immersed and preserved in formalin (10 %) for histopathology examination.

### Experiment-9: Effect of compound of formula (A) on lipopolysaccharides (LPS) induced TNF-α level in NAFLD/NASH mice

To develop NAFLD/NASH model, the same protocol was followed as mentioned in Experiment - 2. After induction, animals were further grouped as follows. Animals fed normal chow diet (n=10) served as a normal control (Group I) and animals fed HFD and HFL were further divided into two groups as disease control (Group II) (n=10) and treatment group (Group III) (n=9). Duration of the treatment was 28 days. During this period Group I and Group II animals were received water as vehicle and Group III animals were treated with compound of formula (A) 100 mg/kg orally. On 27^{th} day, animals were kept for 12 hours fasting and on 28^{th} day, after 60 minutes of treatment, animals were challenged with LPS (500 µg/animal) by intraperitoneal route. Then after 90 minutes of LPS challenge, blood collection was carried out, plasma and serum were separated and stored at - 80 °C in deep freezer for further analysis. Following blood collection, animals were sacrificed; liver, inguinal and epididymal fats were collected and weighed.

### Experiment-10: Effect of disodium salt of compound of formula (C) in HC - HF diet fed Hamsters

The dyslipidemic model was developed in the protocol as mentioned in experimental 4. After 14 days, animals were bled for estimation of the biochemical parameter to confirm induction of disease. After the induction period, the animals were grouped as follows, animals fed normal chow diet (n=6) served as normal control (Group I) and animals fed supranutritional diet with fructose liquid (10 %) were further divided into three groups, as disease control (Group II) (n=7), Group III (n=7) and Group IV (n=7) treated with disodium salt of compound of formula (C) at 10 mg/kg and 30 mg/kg p.o respectively for 28 days. During this period, animal body weight was recorded daily. On 14^{th} and 28^{th} day animals were kept for 4 hours fasting and bled, plasma and serum were separated and used for biochemical analysis.

### Experiment - 11: Effect of compound of formula (A) and disodium salt of compound of formula (C) on NAFLD/NASH in mice

To develop NAFLD/NASH model, the same protocol was followed as mentioned in Experiment - 2. At the end of induction period the animals were grouped as follows. Animals fed normal chow diet (n=8) served as a normal control (Group I) and animals fed HFD and HFL were further divided into three groups (n=9) as NAFLD/NASH control (Group II), Group III and Group IV were dosed with compound of formula (A) and disodium salt of compound of formula (C) at 100 and 30 mg/kg p.o respectively for 28 days. On 28^{th} day, animals were kept for 12 hours fasting and on 29^{th} day blood was collected, plasma separated and used for biochemical and compound concentration analysis. Following blood collection, animals were sacrificed. Liver was collected and weighed. Liver tissues were used for histopathology examination. Gross pathology of liver was also observed.

### Experiment - 12: Compound of formula (A) and disodium salts of Compound of formula (C) on acute alcohol induced hypertriglyceridemia in mice

Male C57BL/6 mice aged 6-8 weeks, and body weight ranges between 22- 28 g were used for this study. Animals were divided into four groups (n = 8) and fed normal chow feed (Nutri Lab® Rodent, Tetragon Chemie Pvt.Ltd., Bangalore) and drinking water. The Group I and Group II animals were served as a normal and disease control treated with vehicle. Group III (n=8) and Group IV (n=8) animals were treated with compound of formula (A) and disodium salts of compound of formula (C) at 100 and 30 mg/kg p.o respectively for seven days. On 7^{th} day one hour post administration of compounds Group II, Group III and Group IV animals were challenged with 40 % alcohol (10 ml/kg b.w) *p.o.* Eighteen hours post challenge animals were bled, plasma separated and used for biochemical parameter analysis.

### Biochemical parameters

The parameters like Glucose, ALT, AST, TC, TG (Clinical chemistry analyzer Erba XL 300), insulin (Linco ® - CA, Product code: EZRMI-13K) and TNFα (GE Healthcare, Amersham, UK. Product code: RPN2718) were measured using commercially available kits. NEFA was measured in serum using commercially available NEFA C kit (Product code No: 279-75401, Wako pure chemical industries ltd, Japan).

**Liver triglycerides:** The liver lipid was extracted according to method described by Folch *et al.,* (*Journal of Biological Chemistry,* **1957,** 497) and Purushotham A *et al.,* (*Journal of Lipid Research,* **2007**,*48*, 444-452). TG was estimated by using standard commercially available kit.

### Liver histopathology:

Liver samples were fixed with 10% formalin and embedded in paraffin. Sections measuring 5 µm were cut and stained with hematoxylin and eosin (HE). Liver histology was examined using the analysis system (NIKON, ECLIPSE- E200, Japan). (Carson FL.,1996).

The animals treated with Compound of formula (A) showed reduction in body weight and fasting blood glucose and hepatic steatosis. The effect of this compound on these features is better than other standard compounds like Metformin, Viladagliptin and Rosiglitazone.

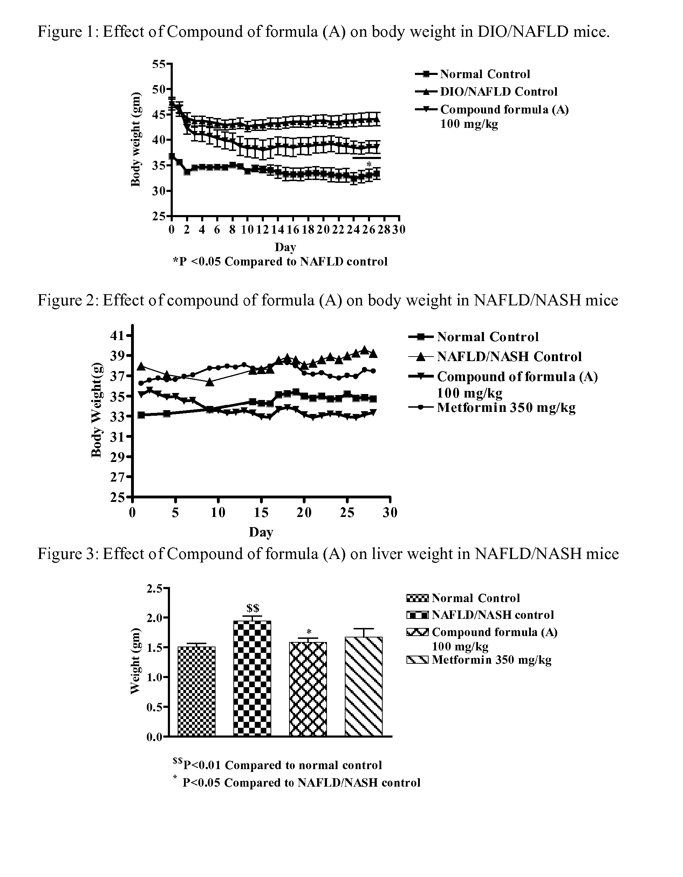

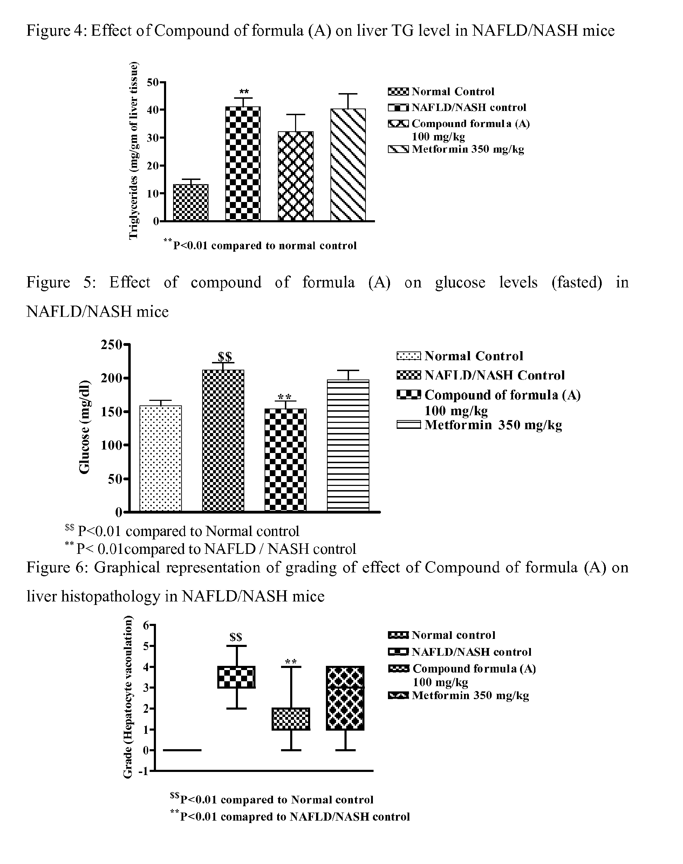

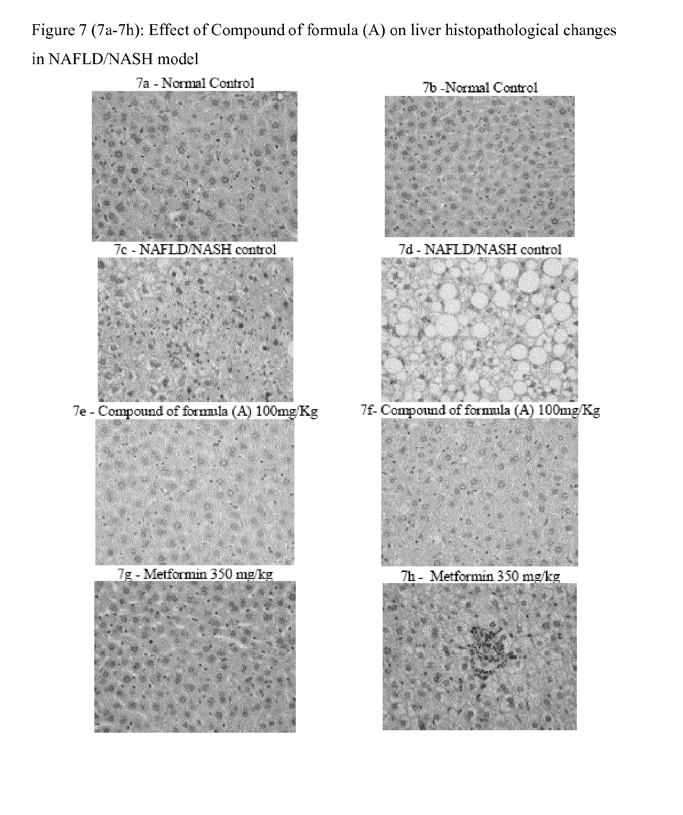

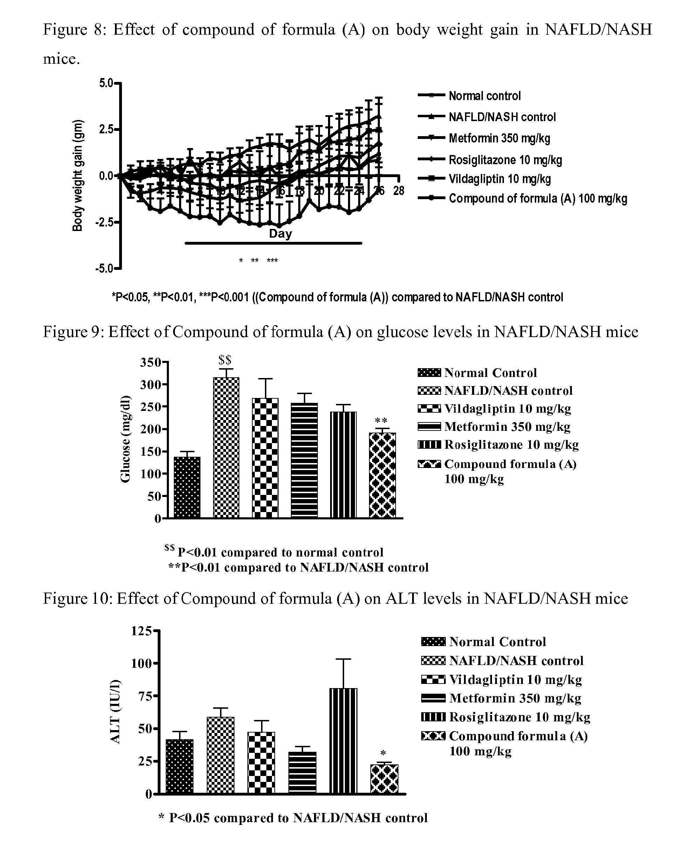

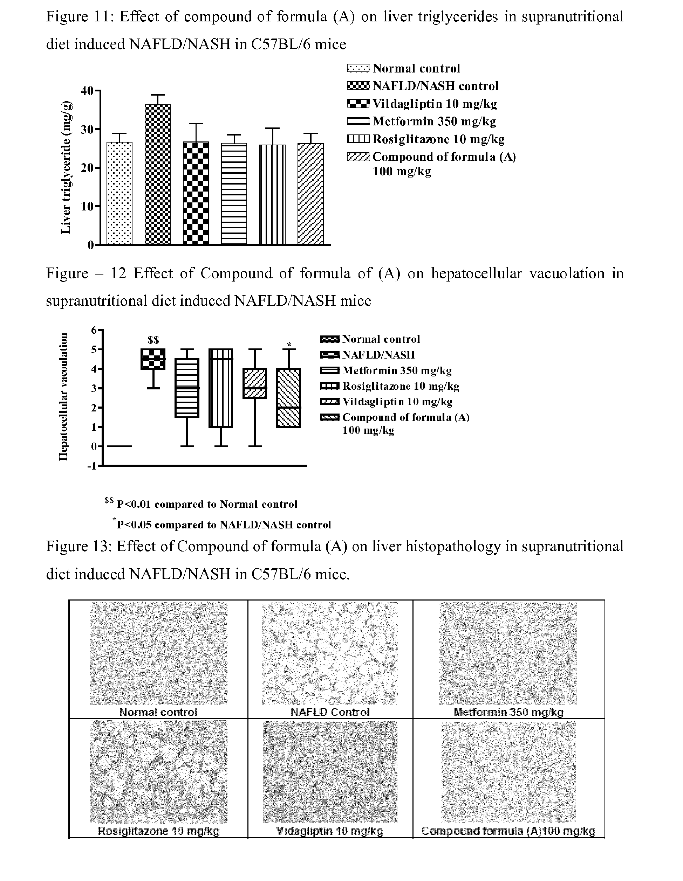

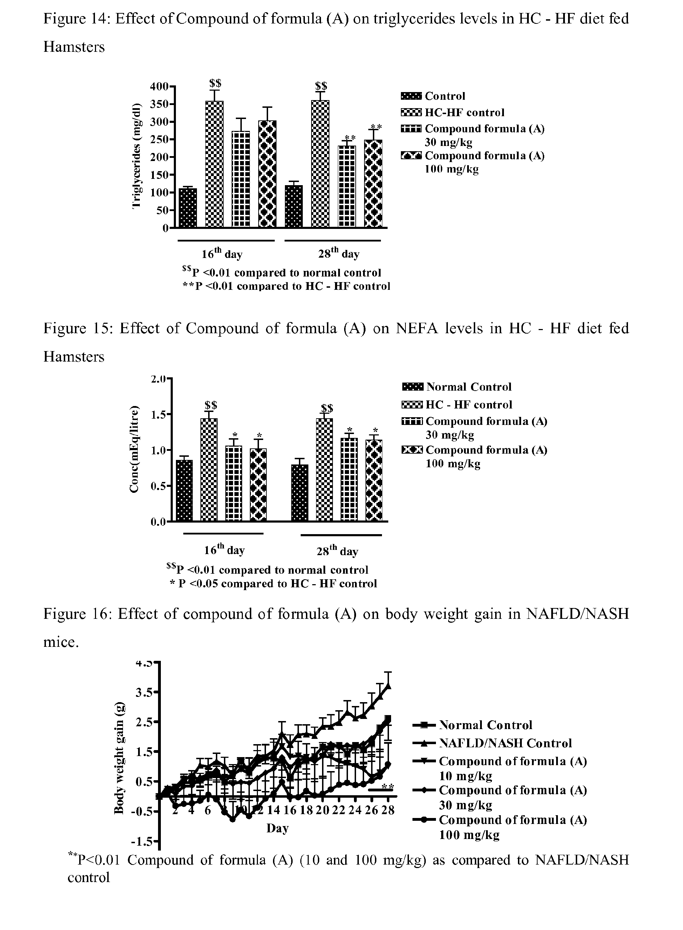

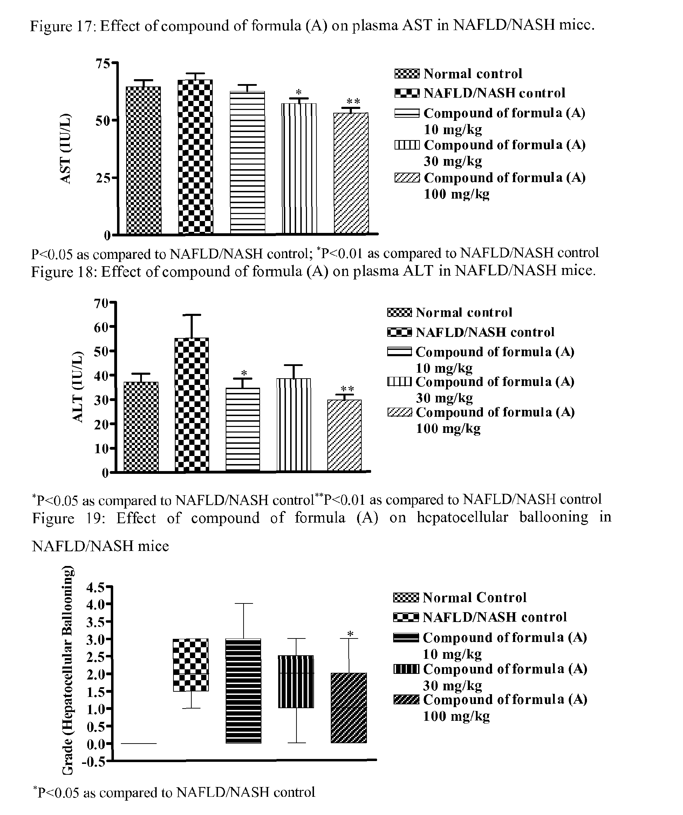

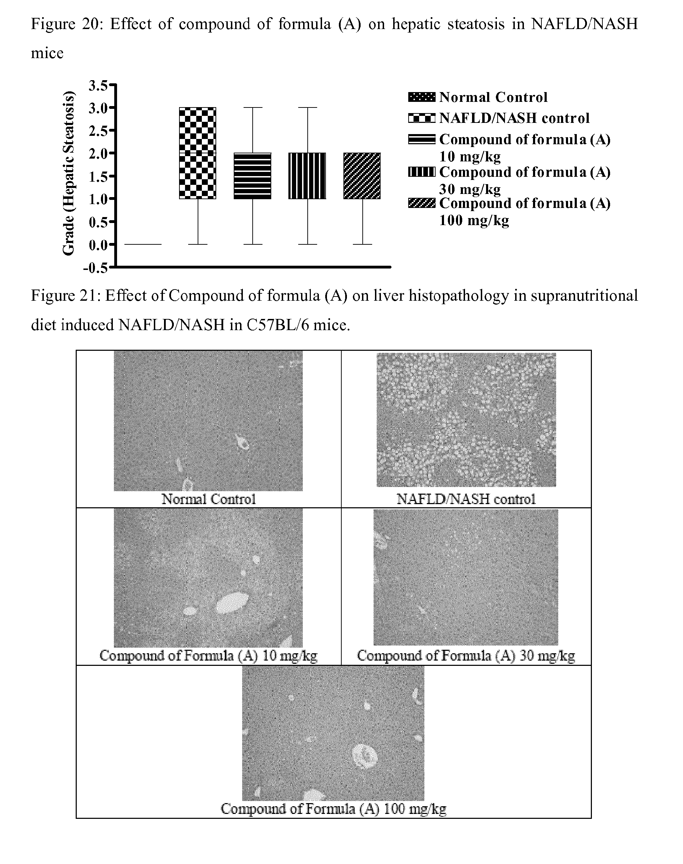

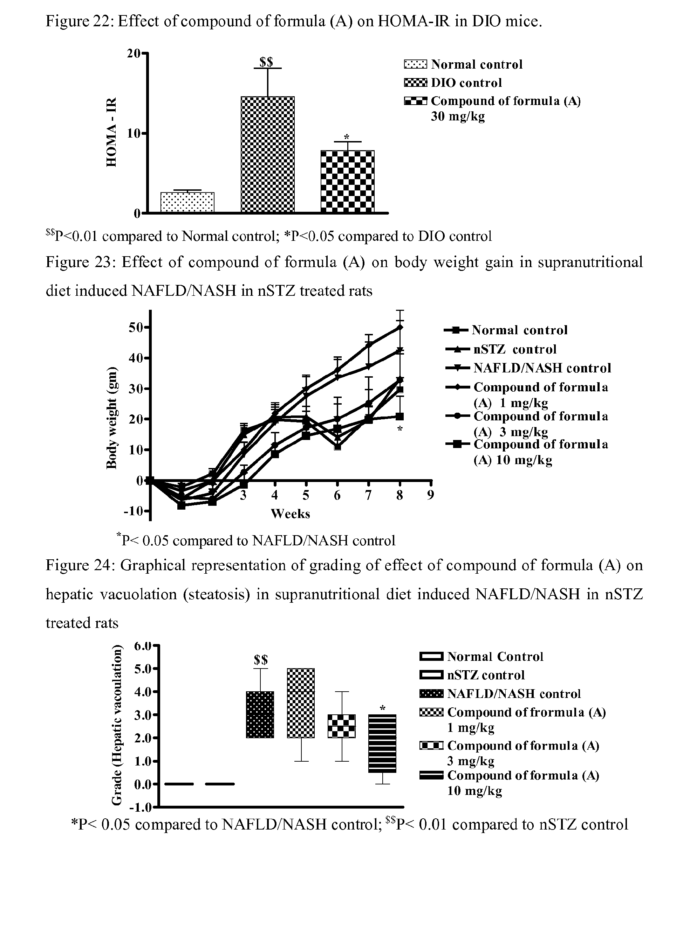

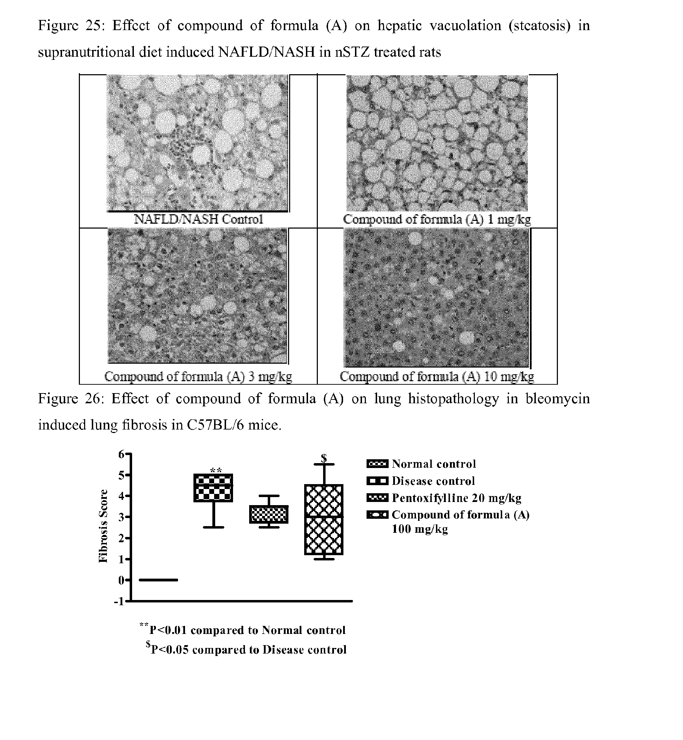

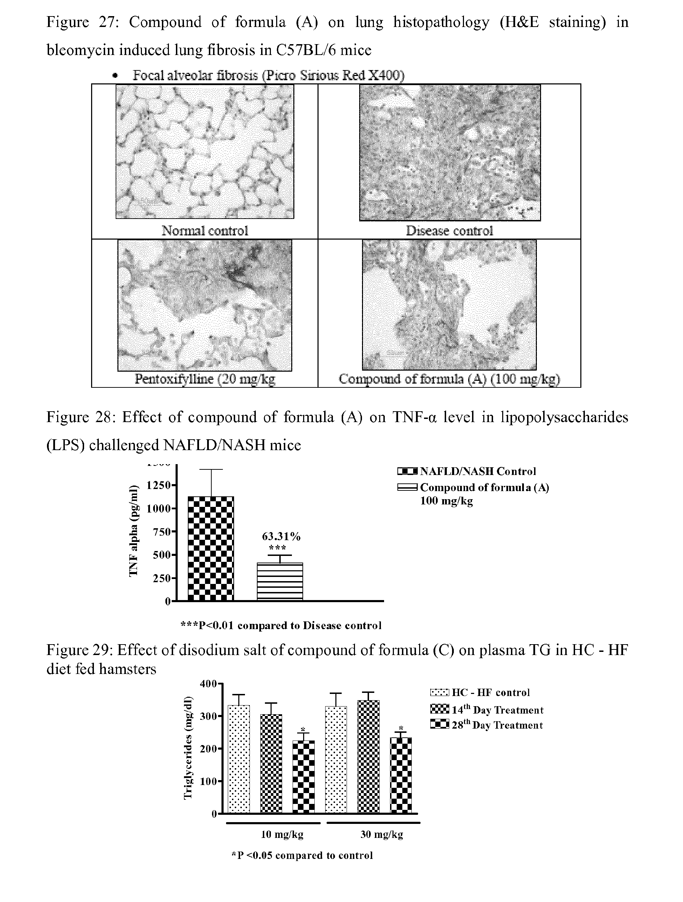

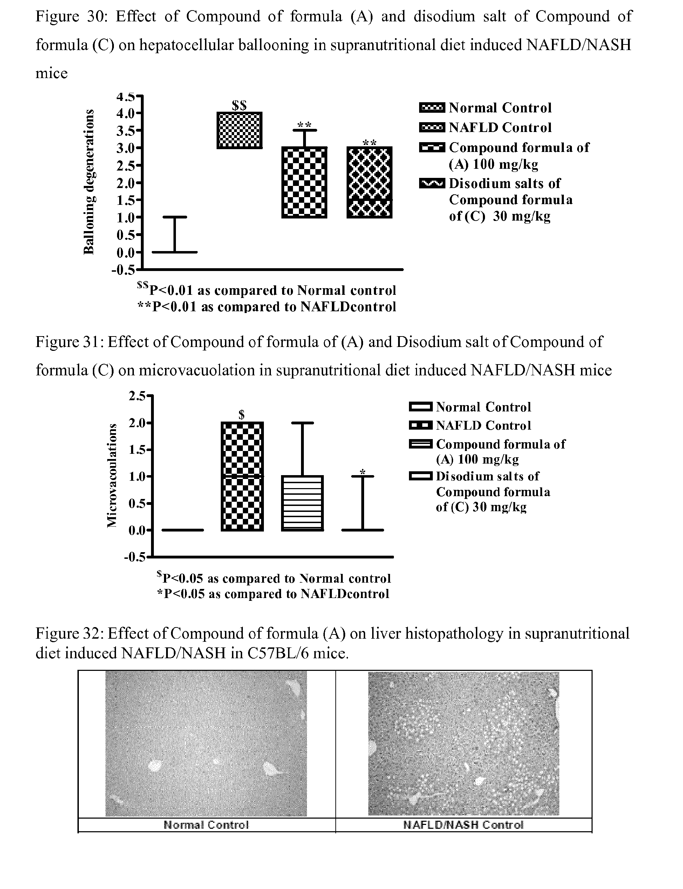

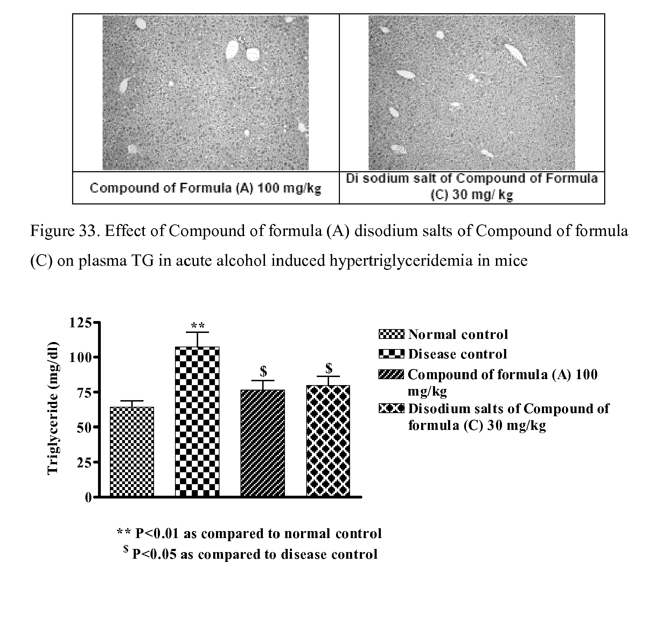

The disease models treated with compound of formula (A) and disodium salts of compound of formula (C) showed significant impact on key features of NAFLD/NASH. Specifically mice exhibited reduction in body weight (Figures 1, 2, 8, 16 & 23), glucose (Figures 5 & 9), AST (Figure 17), ALT (Figures 10 & 18), TNF-α levels (Figure. 28), improvement in HOMA - IR (Figure 22) and improvement in liver histopathology (Hepatic steatosis) (Figures 6, 7, 12, 13, 19, 20, 21, 24, 25, 30, 31 & 32) as compared to relevant untreated disease control. The dyslipidemic hamster treated with compound of formula (A) and disodium salts of compound of formula (C) showed significant reduction in TG and NEFA (Figures 14, 15 & 29). In case of bleomycin induced fibrosis, the compound of formula (A) on treatment significantly prevents fibrosis formation (Figures. 26 & 27). In acute alcohol induced hypertriglyceridemic mice treated with compound of formula (A) and disodium salts of compound of formula (C) showed significant reduction in TG (Figure 33). The effect of this compound on these features is better than other standard compounds like Metformin, Vildagliptin and Rosiglitazone.

Taken together compound of formula (A) has shown remarkable effect on hepatic steatosis the hall mark of NAFLD/NASH, in addition to effect on other key features like obesity, insulin resistance, dyslipidaemia and fibrosis. This compound may be an attractive therapy for the treatment of NAFLD/NASH.

### Data Analysis:

The values are expressed as Mean ± SE. The Graphs were generated using GraphPad Prism® (Version 4). Statistical analysis was undertaken using t-test or One-way ANOVA with Dunnett's post-test or Two- way ANOVA with Bonferroni post test. The results were considered significant when P < 0.05.

The compound of formula (A) also has a significant effect on proinflammatory cytokines like TNF-α and hence are useful in the treatment of diseases like psoriasis.

### Experiment - 13: Effect of Compounds of Formula (A), (B) and (C) on adipogenesis in 3T3-L1 mouse fibroblast

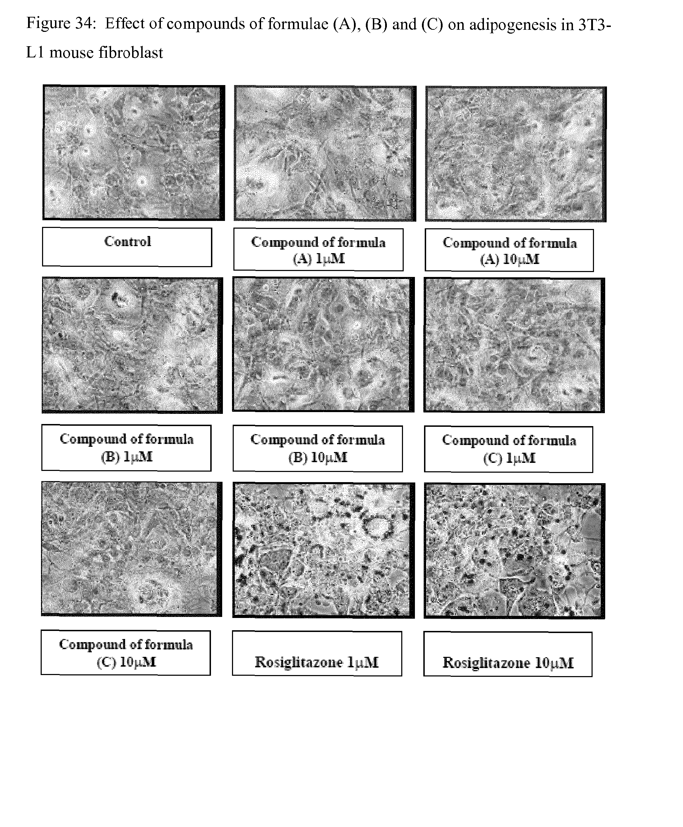

3T3-L1 fibroblasts were seeded at a concentration of 10,000 cells/well in 24 well tissue culture plates in a total volume of 1 mL Dulbecco's Modified Eagle Medium (DMEM media) containing 10 % fetal bovine serum, penicillin and streptomycin. After overnight incubation at 37 °C in a CO₂ incubator, the cells were treated with vehicle (0.01 % DMSO), rosiglitazone (1 µM, 10 µM) and different concentrations of compound of formula (A) (1 µM, 10 µM), compound of formula (B) (1 µM, 10 µM) and compound of formula (C) (1 µM, 10 µM) to test the adipogenic properties. After every two days, the media contents were replaced with fresh media and the drug. This process was carried out for 7 days. After 7 days of treatment, the cells were fixed in 10 % formalin for 1 hour at 40 °C, stained with oil red O solution for 10 minutes and washed 6 times with 1X PBS to remove the excess stain. The plates were dried and visualized under 40X objective using Nikon E200 microscope and the pictures were taken by Nikon DXM 1200C digital camera. Pictures are presented in Figure 34.

The red pigments observed in the cells treated with rosiglitazone at 1 µM or 10 µM are the intracellular lipids stained with oil red O, indicating adipogenesis (Figure 34, rosiglitazone 1 µM and 10 µM). Compounds of formula (A), (B) and (C) at 1 µM or 10 µM did not induce adipogenesis in 3T3-L1 fibroblasts after 7 days of treatment unlike rosiglitazone (Figure 34, compounds of formula (A), (B) and (C) compared to rosiglitazone). Thus, the data shows that compounds of formula (A), (B) and (C) do not induce adipogenesis in 3T3-L1 fibroblasts.

### Experiment - 14: Effect of compounds of formula (B) and (C) on collagen type 1 secretion in activated hepatic stellate cells (HSC)

HSC cells were treated with the drugs and incubated overnight. The conditioned media was taken out and the secreted proteins were extracted using TCA-acetone method. The cells were harvested for western blot analysis and 1% SDS was added to the sample. Proteins were detected by using 1:500 dilution of polyclonal antibody to collagen type 1 (Abcam; AB292) and 1:1000 dilutions of goat anti-rabbit IgG conjugated to horseradish peroxidase (Bangalore GENEI). The blots were developed by using substrate TMB/H₂O₂. Loading control was performed using the β-actin house keeping gene expression level.

Collagen secretion by activated hepatic stellate cells is markedly decreased on treatment with compounds of formula (B) and (C).

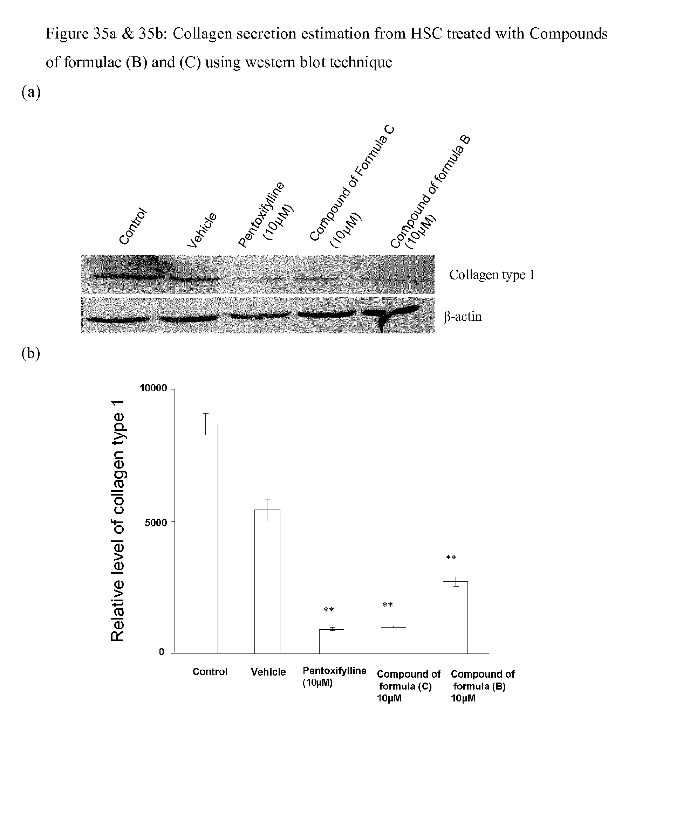

Compounds of the present invention have been shown to inhibit collagen type 1 in activated hepatic stellate cells (Figure 35a & 35b). The effect was comparable to the test compound pentoxifylline at the same concentration. Hence the compounds have anti-fibrotic properties and are useful in the treatment of hepatic fibrosis and other fibrotic diseases wherein the pathology involves modulation of the collagen gene expression (examples: lung fibrosis, renal fibrosis, cardiac fibrosis, scleroderma etc.)

### Experiment-15: Induction of apoptosis in hepatic stellate cells (LX2).

Propidium iodide (PI) has been widely used to measure apoptosis in different experimental systems (Eur. J. Pharmacol., 2003, 473, 117-125; Br. J. Pharmacol., 2009, 158, 1720-1734). The LX2 cells were treated with vehicle (DMSO), staurosporine (0.1 nM), compound of formula (B) (2 µM), compound of formula (C) (2µM) for 8 hours followed by propidium iodide (PI) for 10 minute. Next, cells were washed with PBS for 2 times to remove unbound PI and the fluorescence images were acquired with DP71 camera adapted to an Olympus IX71 microscope. Apoptotic cells were detected by propidium iodide (PI) staining and % apoptotic cells per field were estimated. Number of PI positive cells and total number of cells were counted from the images and plotted as percentage of apoptotic cells per field.

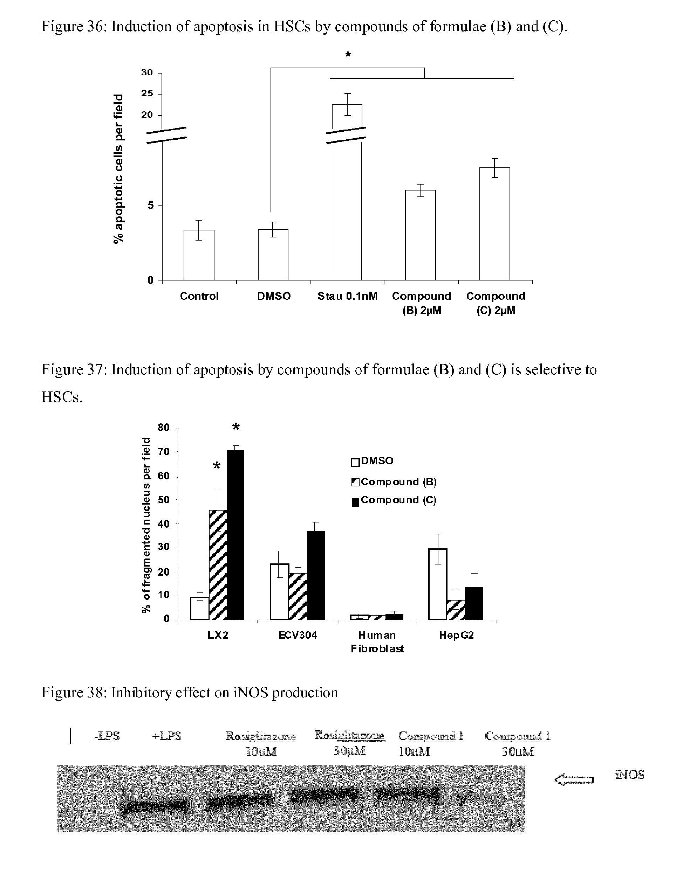

Compounds show significant induction of apoptosis in LX2 cells compared to untreated control or vehicle control. Data showed increase in apoptosis of HSC under compound (B) and compound (C) treatment. * vs Vehicle (DMSO) (P<0.01). On compound (B) treatment at 2 µM, 6±0.40% of the cells were found to be apoptotic. Whereas, on treatment with compound (C), 7.5±0.64% of LX2 cells were apoptotic. These values were significantly higher than the apoptosis induced in vehicle (DMSO) treated cells. In this experiment, staurosporine (stau), a known potent inducer of apoptosis, showed approximately 23% apoptosis in LX2 cells. Therefore, data from the study shows that the compound of formula (B) and compound of formula (C) induce apoptosis in HSCs. The results are provided in Figure 36.

### Experiment-16: Selective induction of apoptosis in hepatic stellate cells (LX2).

The methodology adopted is same as in Experiment-7 above. The results are shown in Figure 37.

Apoptotic cells were detected by propidium iodide (PI) staining and % apoptotic cells per field were estimated. Data showed significant increase in apoptosis of hepatic stellate cells (LX2) under compound (B) and compound (C) treatment. However, no significant apoptosis was observed in human endothelial cells (ECV 304), human fibroblast cells isolated from skin biopsies or human hepatocytes (HepG2) on treatment with compound of formula (B) or (C) * vs. Vehicle (DMSO) (P<0.01)

Compounds show significant induction of apoptosis in hepatic stellate cells compared to untreated control or vehicle control. However, hepatocytes, human fibroblasts isolated from skin biopsies and epithelial cells treated with either compound of formula (B) or (C) did not show significant apoptosis compared to vehicle control (DMSO). Therefore, data from the study shows that the apoptotic effects of both compound of formula (B) and (C) are selective to HSCs.

Selective induction of hepatic stellate cell apoptosis in the liver can promote or enhance the resolution of liver disease and in particular of liver fibrosis. Thus, the compounds provides methods for treating liver disease by selectively inducing hepatic stellate cell apoptosis in the liver.

### 2. Diabetic Complications

**Aldose Reductase:** Rat lens Aldose reductase is used. Test compound and/or vehicle is preincubated with 3000 µg/ml enzyme (J. Enzyme Inhib. 1993, 7, 249-256; Biol. Pharm. Bull. 1994, 17, 458-459) and 0.2 mM NADPH in phosphate buffer pH 6.2 for 15 minutes at 25 °C. The reaction is initiated by addition of 10 mM DL-glyceraldehyde and incubated for another 20 minutes. Determination of the amount of NADPH remained is read spectrophotometrically. Compounds are screened at 10 µM. Since enzyme activity may change from lot to lot, the concentration used will be adjusted if necessary. The standard reference compound is Quercitin, which is tested in each experiment at several concentrations to obtain a competition curve from which its IC₅₀ is calculated (Table 2).

**Table 2: Aldose reductase inhibitory activity**

| S. No | Compound of formula | IC₅₀ (µM) |
|---|---|---|
| 1 | A | 4.07 |
| 2 | B | 4.92 |
| 3 | C | 1.43 |
| 4 | Quercitin | 0.35 |

Based on the results obtained in the *in-vitro* studies, it can be concluded that Compounds A, B and C are moderately potent inhibitors of Aldose Reductase. The IC₅₀ values are comparable to Sorbinil (2 µM) suggestive of potential activity *in-vivo* for the inhibition of aldose reductase and potential treatment for diabetic complications (Biochemical Pharmacology, 1986, 35, 2955-2959).

### iNOS Inhibition:

Compound of formula (A) or Rosiglitazone were incubated first for one hour and then challenged with LPS at 1 µg/mL for 6 hours. At the end of the assay, cell lysates were probed with anti iNOS antibody.

Compound of formula (A) inhibits the iNOS production in these cells (Figure 38). Based on the results, it is concluded that Compound of formula (A) is a potent inhibitor of iNOS with activity superior to Rosiglitazone at equal concentrations (30 µM). Since iNOS is implicated in the pathology of diabetic complications, it is anticipated that Compound of formula (A) will have a protective effect in the development of neovascularization contributed by elevated iNOS.

### Inhibits LPS induced TNF- α and IL-6 Inhibition In Vivo:

This study was undertaken when the mice are at 10-11 weeks in age. On Day 0 their body weights were measured and they were assigned to two groups of 6 mice each to get similar average group body weights. They were orally administered with either vehicle (deionized water, Group 1) or Compound of formula (A) (50 mg/kg; Group 2) daily between 9:30 and 10:30 AM for 10 days. The body weights were measured on day 3, 7 and 10 of the study before administration of vehicle or drug. On Day 10, the mice were administered LPS at 400 µg/kg, IP. After 1 hour they were orally administered with either vehicle or Compound of formula (A). After another 90 minutes, the mice were euthanized by carbon dioxide asphyxiation. Blood was collected by cardiac puncture and was processed to obtain serum. The sera were stored at -80 °C for *ex vivo* analysis of TNF-α and IL-6 cytokines.

### Serum TNF- α Immunoassay:

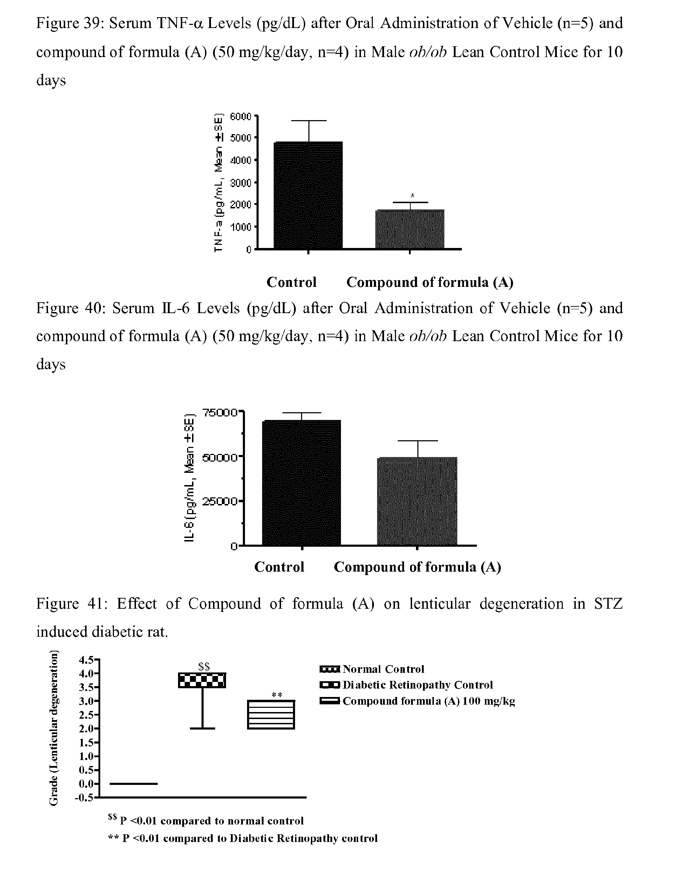

Serum TNF-α concentration for each group of mice is determined using the Quantikine. Mouse TNF-α Immunoassay kit. The manufacturer's instructions were followed for the assay. The serum samples from both the vehicle and Compound of formula (A) treated groups of mice were diluted 1:20 in Calibrator Diluent. The optical density of each well was read at 450 nm using the Microplate Reader (Figure 39).

### Serum IL-6 Immunoassay:

Serum IL-6 concentration for each group of mice is determined using the Quantikine Mouse IL-6 Immunoassay kit. The manufacturer's instructions were followed for the assay. The serum samples from the vehicle-treated group of mice were diluted 1:200 in Calibrator Diluent. The serum samples from the treatment group of mice were diluted 1:100 in Calibrator Diluent. The optical density of each well was read at 450 nm Microplate Reader (Figure 40).

### Improved Lenticular degeneration in Streptozotocin (STZ) induced type I diabetic model in vivo:

The animal model was developed by injecting STZ to Sprague Dawley rats and kept for 10 weeks to develop diabetic complications. Initially animals were in hyperglycemic and chronically, developed several feature of diabetes and diabetic complications, which simulates human diabetes and complications. These features were treated as mentioned below. Forty male Sprague Dawley rats aged 6 - 8 weeks, body weight range between 150-190 g was used for this study. The animals were divided into two groups. First group (n = 10) was treated as a normal control injected with normal saline and the second group (n = 30) was treated with STZ (50 mg/kg, i.v.), (Sigma. USA). Body weight was recorded every week and blood glucose every month using glucometer (Contour TS, Bayer Healthcare Ltd.). Every week eyes were examined for cataractous changes. After 10 weeks of diabetes induction animals were grouped based on blood glucose levels and the severity of cataract. The first group served as normal control (n=10). The diabetic animals with cataract were divided into two groups, second group served as disease control (n= 9) and third group was treated with compound of formula (A) (n=10) at 100 mg/kg dose for 2 months. At the last day of experiment animals were kept for 12 hours fasting, next day blood was collected, plasma separated and used for biochemical analysis. The animals were then sacrificed; both the eyes were collected and used for histopathology examination.

Oral administration of the compound of formula (A) impacted each of key features of diabetic complications. Specifically mice exhibited reduction in TNF-α (Figure 39) and IL-6 (Figure 40). The rat treated with compound of formula (A) showed significant improvement in lenticular degeneration (Figure 41). In case of in vitro, compound of formula (A) exhibited reduction in aldose reductase activity and iNOS production (Figure 38).

### Data Analysis:

The values are expressed as Mean ± SE. The graphs were generated using GraphPad Prism® (Version 3). Statistical analysis was undertaken by two-tailed unpaired t-test with Welch's correction and 2-Way ANOVA with Bonferroni's post-test using GraphPad Prism®. The results were considered significant when p < 0.05.

### 3. Matrix metalloproteinase - 2 (MMP-2) inhibitory activity (at 10 µM)

MMP-2 enzymes are implicated in lung metastasis and there inhibition is useful to prevent or treat lung metastasis (Journal of Ethnopharmacology 2011, 133, 426-433). The compounds of the present invention are known to inhibit MMP-2 activity and hence useful to prevent or treat lung metastasis (Table 3).

**Table 3: Matrix metalloproteinase-2 inhibitory activity**

| S. No | Compound of formula | % Inhibition |
|---|---|---|
| 1 | (B) | 59 |
| 2 | (C) | 16 |

### Aspects of the invention

1. A Compound of formula (I), their derivatives, analogs, tautomeric forms, stereoisomers, polymorphs, hydrates, solvates, pharmaceutically acceptable salts, pharmaceutically acceptable compositions, metabolites and prodrugs, for use in the prevention or treatment of liver disorder and associated diseases; lung disorder and associated diseases; diabetic complications and cardiovascular diseases comprising administering a therapeutically 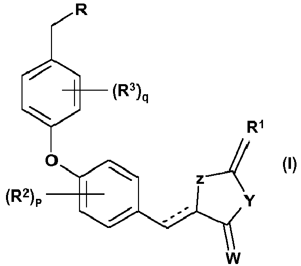 effective amount of compound of formula (I), to a subject in need thereof; whereinrepresents an optional bond; W represents O or S; Y represents NR⁴, S or O; wherein R⁴ represents hydrogen, substituted or unsubstituted groups selected from alkyl, alkenyl and aryl, a counter ion or -CH₂COR⁶; wherein R⁶ represents -OH, -NH₂, -NHOH or OR¹⁸; wherein R¹⁸ is an alkyl group; Z represents -CR⁵ or S; R¹ represents O, S or together with R⁵ forms a fused 5 or 6 membered aromatic or heteroaromatic ring system containing carbon atoms or 1 or 2 heteroatoms selected from O, S or N;
   R², R³ and R⁵ independently represent hydrogen, halogens, hydroxy, nitro, cyano, formyl, amino, alkyl, haloalkyl or alkoxy;
   R represents either of U or V; wherein U represents hydrogen, halogen, hydroxy, nitro, cyano, formyl, amino, -COR¹⁰ or substituted or unsubstituted groups selected from linear or branched (C₁-C₆) alkyl group and (C₁-C₆) alkoxy group; R¹⁰ represents -OR¹¹ or -NR¹²R¹³; wherein R¹¹ represents hydrogen, substituted or unsubstituted groups selected from alkyl, alkenyl, aryl, aralkyl and heteroaryl or a counter ion; R¹² and R¹³ independently represent hydrogen, substituted or unsubstituted groups selected from alkyl, alkenyl, aryl and heteroaryl; or R¹² and R¹³ together form a heteroaliphatic or heteroaromatic ring;
   V represents 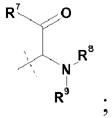 R⁷ represents -OR¹⁴, wherein R¹⁴ represents hydrogen, substituted or unsubstituted groups selected from alkyl, alkenyl, aryl, aralkyl and heteroaryl or a counter ion; or -NR¹⁵R¹⁶; wherein R¹⁵ and R¹⁶ independently represent hydrogen or substituted or unsubstituted groups selected from alkyl, alkenyl, aryl and heteroaryl; R⁸ and R⁹ independently represent hydrogen, substituted or unsubstituted groups selected from alkyl, alkenyl, aryl and heteroaryl or -COR¹⁷; wherein R¹⁷ represents substituted or unsubstituted groups selected from alkyl, aryl, heteroaryl, alkenyl, alkenyloxy, aryloxy, alkoxy and aralkoxy;
   p and q are integers selected from 1-3.
2.Compound for use according to aspect 1, wherein the compound of formula (I) is selected from
   i.Methyl (2*S*)-2-amino-3-(4-{4-[(2,4-dioxo-1,3-thiazolidin-5-yl)methyl] phenoxy}phenyl)propanoate or its salt;
   ii.(2*S*)-2-Amino-3-(4-{4-[(2,4-dioxo-1,3-thiazolidin-5-yl)methyl]phenoxy} phenyl)propanoic acid or its salt and
   iii.(4-{4-[(2,4-Dioxo-1,3-thiazolidin-5-yl)methyl]phenoxy}phenyl)acetic acid or its salt.
3.Compound for use according to aspect 2, wherein the said salt is selected from hydrochloride, hydrobromide, sodium, potassium or magnesium salt.
4.Compound for use according to aspect 1, wherein the compound is hydrochloride salt of Methyl (2*S*)-2-amino-3-(4-{4-[(2,4-dioxo-1,3-thiazolidin-5-yl)methyl]phenoxy} phenyl) propanoate.
5.Compound for use according to aspect 1, wherein the compound is hydrochloride salt of (2*S*)-2-Amino-3-(4-{4-[(2,4-dioxo-1,3-thiazolidin-5-yl)methyl]phenoxy}phenyl) propanoic acid.
6.Compound for use according to aspect 1, wherein the compound is (4-{4-[(2,4-Dioxo-1,3-thiazolidin-5-yl)methyl]phenoxy}phenyl)acetic acid.
7.Compound for use according to aspect 1, wherein the compound is disodium salt of (4-{4-[(2,4-Dioxo-1,3-thiazolidin-5-yl)methyl]phenoxy}phenyl)acetic acid.
8.Compound for use according to aspect 1, wherein the liver disorder is selected from Non-Alcoholic Fatty Liver Disease (NAFLD), Non-Alcoholic Steatohepatitis (NASH), hepatic fibrosis, liver cirrhosis and alcoholic steatohepatosis.
9.Compound for use according to aspect 1, wherein the liver disorder is NAFLD and NASH.
10. Compound for use according to aspect 1, for treating NAFLD and NASH.
11. Compound for use according to aspect 1, wherein the diabetic complication is a macro complication selected from ischemic heart disease, cerebrovascular disease and peripheral vascular disease or a micro complication selected from cataract, retinopathy, nephropathy, neuropathy, maculopathy and glaucoma.
12. Compound for use according to aspect 1, wherein the cardiovascular disease is selected from atherosclerosis, restenosis, hypertension, vasospasm, and cardiac hypertrophy.
13. Compound for use according to aspect 1, wherein the associated diseases are selected from psoriasis, scleroderma, lung metastasis, lung fibrosis, polycystic ovary syndrome and obstructive apnoea.
14. Compound for use according to aspect 1, wherein diabetic complications are selected from diabetic retinopathy, diabetic nephropathy, diabetic neuropathy and diabetic cataract.
15. Compound for use according to aspect 1, for reducing plasma or liver triglycerides levels.
16. Compound for use according to aspect 1, for reducing plasma glucose levels.
17. Compound for use according to aspect 1, for inhibiting TNF-α, IL-6, iNOS, aldose reductase and matrix metalloproteinase-2.
18. A pharmaceutical composition comprising a compound of formula (I) as an active ingredient, along with a pharmaceutically acceptable carrier to treat liver disorder and associated diseases; lung disorder and associated diseases; diabetic complications and cardiovascular diseases according to aspect 1 or aspect 2.
19. Compound of formula (I) for use as mentioned in aspects 2-6, substantially as hereinbefore described or exemplified.
20. Compound of formula (I) for use as mentioned in aspects 1, 8, 9, 10, 11, 12, 14, 15 and 16, substantially as herein described with reference to the examples and/or the figures of the accompanying drawings.

## Claims

1. A Compound of formula (I), their derivatives, analogs, tautomeric forms, stereoisomers, polymorphs, hydrates, solvates, pharmaceutically acceptable salts, pharmaceutically acceptable compositions, metabolites and prodrugs, for use in the prevention or treatment of lung disorder and associated diseases: 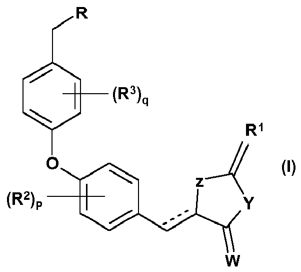 wherein ---- represents an optional bond; W represents O or S; Y represents NR⁴, S or O; wherein R⁴ represents hydrogen, substituted or unsubstituted groups selected from alkyl, alkenyl and aryl, a counter ion or -CH₂COR⁶; wherein R⁶ represents -OH, -NH₂, -NHOH or OR¹⁸; wherein R¹⁸ is an alkyl group; Z represents -CR⁵ or S; R¹ represents O, S or together with R⁵ forms a fused 5 or 6 membered aromatic or heteroaromatic ring system containing carbon atoms or 1 or 2 heteroatoms selected from O, S or N;
R², R³ and R⁵ independently represent hydrogen, halogens, hydroxy, nitro, cyano, formyl, amino, alkyl, haloalkyl or alkoxy;
R represents either of U or V; wherein U represents hydrogen, halogen, hydroxy, nitro, cyano, formyl, amino, -COR¹⁰ or substituted or unsubstituted groups selected from linear or branched (C₁-C₆) alkyl group and (C₁-C₆) alkoxy group; R¹⁰ represents -OR¹¹ or -NR¹²R¹³; wherein R¹¹ represents hydrogen, substituted or unsubstituted groups selected from alkyl, alkenyl, aryl, aralkyl and heteroaryl or a counter ion; R¹² and R¹³ independently represent hydrogen, substituted or unsubstituted groups selected from alkyl, alkenyl, aryl and heteroaryl; or R¹² and R¹³ together form a heteroaliphatic or heteroaromatic ring;
V represents 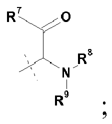 R⁷ represents -OR¹⁴, wherein R¹⁴ represents hydrogen, substituted or unsubstituted groups selected from alkyl, alkenyl, aryl, aralkyl and heteroaryl or a counter ion; or -NR¹⁵R¹⁶; wherein R¹⁵ and R¹⁶ independently represent hydrogen or substituted or unsubstituted groups selected from alkyl, alkenyl, aryl and heteroaryl; R⁸ and R⁹ independently represent hydrogen, substituted or unsubstituted groups selected from alkyl, alkenyl, aryl and heteroaryl or -COR¹⁷; wherein R¹⁷ represents substituted or unsubstituted groups selected from alkyl, aryl, heteroaryl, alkenyl, alkenyloxy, aryloxy, alkoxy and aralkoxy;
p and q are integers selected from 1-3.

2. Compound for use according to claim 1, wherein the compound of formula (I) is selected from
i.Methyl (2*S*)-2-amino-3-(4-{4-[(2,4-dioxo-1,3-thiazolidin-5-yl)methyl] phenoxy}phenyl)propanoate or its salt;
ii.(2*S*)-2-Amino-3-(4-{4-[(2,4-dioxo-1,3-thiazolidin-5-yl)methyl]phenoxy} phenyl)propanoic acid or its salt and
iii.(4-{4-[(2,4-Dioxo-1,3-thiazolidin-5-yl)methyl]phenoxy}phenyl)acetic acid or its salt.

3. Compound for use according to claim 2, wherein the said salt is selected from hydrochloride, hydrobromide, sodium, potassium or magnesium salt.

4. Compound for use according to claim 1, wherein the compound is hydrochloride salt of Methyl (2*S*)-2-amino-3-(4-{4-[(2,4-dioxo-1,3-thiazolidin-5-yl)methyl]phenoxy} phenyl) propanoate.

5. Compound for use according to claim 1, wherein the compound is hydrochloride salt of (2*S*)-2-Amino-3-(4-{4-[(2,4-dioxo-1,3-thiazolidin-5-yl)methyl]phenoxy}phenyl) propanoic acid.

6. Compound for use according to claim 1, wherein the compound is (4-{4-[(2,4-Dioxo-1,3-thiazolidin-5-yl)methyl]phenoxy}phenyl)acetic acid.

7. Compound for use according to claim 1, wherein the compound is disodium salt of (4-{4-[(2,4-Dioxo-1,3-thiazolidin-5-yl)methyl]phenoxy}phenyl)acetic acid.

8. Compound for use according to claim 1, wherein the associated diseases are selected from psoriasis, scleroderma, lung metastasis, lung fibrosis, polycystic ovary syndrome and obstructive apnoea.
